# EUROPEAN PATENT APPLICATION

(11) **EP 3 859 331 A1**
(43) Date of publication of application: **04.08.2021**
(21) Application number: 20155033.2
(22) Date of filing: 31.01.2020
(51) Int. Cl.: G01N 33/50, G01N 33/68

(54) **METHODS FOR ASSIGNING A PHENOTYPIC SIGNATURE FOR DIAGNOSTIC AND THERAPEUTIC APPLICATIONS**

(71) Applicant: Deutsches Zentrum für Neurodegenerative Erkrankungen e.V. (DZNE), 53127 Bonn (DE)
(72) Inventor: FAVA, Eugenio, 53129 Bonn (DE); DENNER, Philip, 53177 Bonn (DE); MÖHL, Christoph, 53125 Bonn (DE); KURKOWSKY, Birgit, 53227 Bonn (DE); ROTH, Wera, 53343 Wachtberg (DE); STAPPERT, Dominik, 53127 Bonn (DE); SCHNEIDER, Anja, 53123 Bonn (DE); AlSaeedi, Ahmed Hamred, 41464 Neuss (DE)
(74) Representative: Krauss, Jan

(57) **Abstract**

The present invention relates to methods for assigning a phenotypic signature of cells in a liquid biological sample obtained from a mammal, to an innate immune response group using multivariate classification algorithms. Furthermore, the present invention relates to a method for identifying whether a mammal suffers from an inflammation-related disease or is at risk of suffering from an inflammation-related disease using the phenotypic signature. Moreover, the present invention relates to a method for stratifying a mammal suffering from an inflammation-related disease for a treatment against said inflammation-related disease. In addition, the present invention relates to a method for monitoring the progression of an inflammation-related disease in a mammal during a treatment of said mammal. Also, the present invention relates to a method for identifying a compound and/or environmental condition that induces or represses the innate immune response of cells obtained from a mammal. Furthermore, the present invention relates to a use of said phenotypic signature for diagnostic and/or drug de-risking applications.

## Description

### FIELD OF THE INVENTION

The present invention relates to methods for assigning a phenotypic signature of cells in a liquid biological sample obtained from a mammal, to an innate immune response group using multivariate classification algorithms. Furthermore, the present invention relates to a method for identifying whether a mammal suffers from an inflammation-related disease or is at risk of suffering from an inflammation-related disease using the phenotypic signature. Moreover, the present invention relates to a method for stratifying a mammal suffering from an inflammation-related disease for a treatment against said inflammation-related disease. In addition, the present invention relates to a method for monitoring the progression of an inflammation-related disease in a mammal during a treatment of said mammal. Also, the present invention relates to a method for identifying a compound and/or environmental condition that induces or represses the innate immune response of cells obtained from a mammal. Furthermore, the present invention relates to a use of said phenotypic signature for diagnostic and/or drug de-risking applications.

### BACKGROUND

Alzheimer's disease and other dementia are serious morbidities affecting a large number of people worldwide. Those diseases represent social challenge and at the moment no treatment is available. Diagnosis of neurodegenerative disease as well as clinical trial studies have been hindered, in part, by the limited number of biomarker tests that can correctly diagnose these diseases, adequately stratify and enrich cohorts for patients with target pathology, reliably monitor disease progression, and the lack of affordable and non-invasive tests.

There is a growing number of scientific studies where it has been shown that inflammatory processes in the brain are involved in the development of chronic neurodegenerative diseases and that systemic/peripheral immune responses contribute to disease progression. Indeed, the brain does not keep an absolute immune privilege and peripheral inflammatory mediators, such as cytokines, danger signals or other signaling molecules in addition to infiltrating peripheral immune cells can reach the brain and the central nervous system (CNS) (Cunningham et al. 2013, Heppner et al. 2015, McManus and Heneka 2017). Peripheral inflammatory events and infections, such as respiratory tract infections, bacterial, viral and fungal infections are found to be associated with and significantly contribute to the cognitive decline and progression of dementia (McManus and Heneka 2017, Giridharan et al. 2019, Paouri and Georgopoulos 2019). Employing a biocomputational systems level approach based on constructed molecular networks and by the integration of preclinical, genetic and bioinformatic data a strong contribution of immune-regulatory pathways was assigned to late-onset Alzheimer's disease (Zhang et al. 2013).

Up to now, numerous reports have documented altered protein levels of inflammatory mediators in the body fluids, i.e. plasma, serum and CSF of patients with Alzheimer's disease and mild cognitive impairment (MCI) (Brosseron et al. 2014). Although many studies provided controversial or inconclusive results, they provide evidence that alterations of immunological parameters coupled to Alzheimer's disease are reflected in the periphery. Furthermore, meta-analyses provided evidence to support that systemic inflammation might be a biomarker for AD diagnosis (Su et al. 2019).

In the context of innate immunity, many recent studies provided evidence for a potential relationship of inflammasome activation and inflammation-related diseases, in particular neurological disease (Lang et al. 2018). A key player in mediating inflammatory responses in many complex age-related and inflammatory diseases including neurodegenerative disease and AD is the cytosolic innate immune signaling receptor NLRP3 (NLR-family pyrin domain containing 3) (Mangan et al. 2018). It senses pathogen- and danger-associated molecular pattern- (PAMP- and DAMP-) derived signals and assembles upon activation a multiprotein inflammasome complex leading to caspase-i mediated proteolytic activation and release of cytokines interleukin-ibeta (IL-ibeta) and IL-18 and subsequent pyroptotic cell death. Chronic activation of the NLRP3 inflammasome triggering persistent neuroinflammation is emerging as a crucial contributing pathomechanism to the development and progression of AD (Heneka et al. 2015, Heneka et al. 2013, Halle et al. 2008) and was found in microglia to be connected to seeding and spreading of amyloid-beta pathology in an AD mouse model (Venegas et al. 2017).

Furthermore, latest evidence was provided in an AD mouse model that peripheral immune challenges could induce microglial changes resulting in a reduced microglial clearance capacity of Abeta (Tejera et al. 2019). Recently in the human system, increased expression of NLRP3 inflammasome components were found in monocytes of AD patients accompanied by increased production of cytokines IL-ibeta and IL-18 (Saresella et al. 2016). Together, these studies provide evidence that the activation of the Nlrp3 inflammasome contributes to AD pathology in the brain and that pathological changes may manifest and become detectable in peripheral blood.

For inflammation-related diseases and in particular Alzheimer's disease CSF biomarkers are successfully used in clinical diagnosis with high accuracy. However, there is the demanding need for a minimally invasive, cost-effective and reliable method to diagnose early-stage Alzheimer's disease (Blennow 2017, Zetterberg and Burnham 2019).

Recently, ultrasensitive immunoassays have been developed based on immunoprecipitation-mass spectrometry and have successfully been used to measure plasma Abeta biomarkers and to discriminate between healthy controls and patients with AD or mild cognitive impairment (Nakamura et al. 2018). The authors could demonstrate that the assessment of plasma APP/Abeta and Abeta40/42 ratios allow to accurately predicting brain Abeta burden (Nakamura et al. 2018, Baldacci et al. 2019, Zetterberg 2019).

Beyond that, it was recently shown that high-precision plasma Abeta42/40 ratio could accurately diagnose brain amyloidosis and therefore can be used to screen cognitively normal individuals to identify at-risk individuals for AD dementia (Schindler et al. 2019, Bendlin et al. 2019). Moreover, plasma neurofilament light chain (NfL) emerged in recent times as a reliable peripheral biomarker of neurodegenerative disease (Baldacci et al. 2019, Zetterberg 2019) showing a good diagnostic accuracy in differentiating neurodegenerative patients from controls and starting from early stages of disease progression (Khalil et al. 2018). Indeed Preische et al. could demonstrate that CSF NfL and serum NfL levels correlated and were found to be elevated at pre-symptomatic stages of familial Alzheimer's disease (Preische et al. 2019), Thus, serum NfL assessment could predict disease progression and brain neurodegeneration at the early pre-symptomatic stages of familial AD. Altogether these facts indicate that blood-based biomarker tests can be possible.

However, there is a huge need for methods that can be used to detect reliable changes, a combination and the analysis of multiple parameters is a necessary step which is supported by the recent expert review by Penner et al. considering biomarkers in blood as a "shadow of events that are occurring in the brain" and expressed as a "fingerprint in blood" worthwhile to follow up in order to overcome the constraint in biomarker development (Penner et al. 2019).

In recent years, a number of multivariate blood-based biomarker panels have been tested and proposed for diagnosing, classifying and prognosing AD, following the hypothesis that the use of panels of markers may outperform single candidate markers (Zetterberg and Burnham 2019). However, overall there has been a lack of harmonization and replication of the findings (Zetterberg and Burnham 2019). Lately, a hypothesis-led plasma biomarker search was conducted and 53 inflammatory proteins were analyzed in healthy controls, MCI and AD individuals and a collection of analytes and markers of inflammation and complement dysregulation showed significant intergroup differences which could be replicated in an independent cohort (Morgan et al. 2019).

Finally, the Korean AD Research Platform Initiative based on Immune-Inflammatory biomarkers (K-ARPI) has launched a combinatorial approach in order to develop novel biomarkers for AD, to stratify subpopulations of patients with AD and to develop new drug candidates for delaying the progression of AD by modulating toxic immune inflammatory response (Kim et al. 2019). This strategy was based on a decision platform integrating pre-screening of patients by clinical characteristics, genetic information, cell- and image-based readouts and cytokine/immune modulator profiling, and to be combined with a computational layer of data analysis employing classification algorithms and multimodal deep learning programs. However, all methods are not particularly robust, since especially biomarkers in the blood can fluctuate due to various influences or are associated with considerable effort and/or high costs.

Apart from that, in the field of drug development, drug de-risking, drug safety and risk management play a major role. The financial factor is enormous. It is estimated that one in every 10,000 molecules entering drug research makes it to the market - and even then there is no guarantee that the expensive development costs will be recovered. Apart from the financial loss that a company has to bear, patient safety naturally plays an even greater role. A newly developed drug can fail in the clinical phases, and/or lead to unforeseeable side effects, or even trigger inflammation in patients. In addition, approved drugs also may cause inflammation in patients, due to individual intolerances depending on the patient's individual immune system. Therefore, the interest in developing respective modern diagnostic methods is increasing.

More and more, drugs are to be used in a personalized manner in order to be able to exploit the individual modes of action of a drug in a patient more specifically, and thus to avoid inflammatory reactions. Suitable biomarkers are being sought that allow for a reliable statement that can be made as to whether the drug under consideration is likely to be effective in this patient; whether the specific patient is likely to tolerate the drug under consideration; and how the drug is best dosed for this patient.

Overall, there is a lack of methods for obtaining a robust, reliable, fast, inexpensive and convenient diagnosis of inflammatory diseases, at the same time allowing the stratification of the patients and subsequent monitoring of the therapy can be carried out, so that potentially inflammation-triggering drugs can be ruled out in advance through drug de-risking.

A considerable challenge of personalized medicine is the analysis of the diagnostic data obtained. For example, genetic data - obtained from procedures such as next-generation sequencing - require computationally complex data processing steps before the actual analysis of the data can take place. The subsequent analysis then requires interdisciplinary cooperation between experts from the medical profession, clinical oncologists, biologists and software engineers. The provision of personalised therapies is therefore associated with a high diagnostic effort.

Currently, automated workflows, disclosed in the prior art are optimized for homogenous readouts or for image-based readouts in human cell lines or murine cell models. These approaches are not able to link intracellular processes on single cell level with extracellular signals of the innate immune response, but this is essential to understand and evaluate the mode of action of perturbation effects in predictive drug discovery and de-risking innate immunity.

Nakahata et al. disclose a method for screening drugs for suppressing inflammasome activity, using macrophages derived from induced pluripotent stem cells having mutant NLRP3 gene and contacting them with LPS, as an external stimulus, and optionally a drug of interest, wherein the Il1-beta secretion of contacted and naive cells is analysed. [4]

Jankovic et al. show a method for the prevention or treatment of acne, comprising the administration of an inhibitor capable of binding to a member of the inflammasome group consisting of IL-1β, IL-1 receptor type 1, NLRP3, ASC, caspase-i and cathepsin B, said inhibitor being selected from *inter alia* an antibody, an antibody fragment, and an antibody-like molecule [5].

The prior art discloses a number of approaches for obtaining and evaluating quantitative data from microscopic imaging.

Loo et al. disclose a multivariate method for the classification of untreated and treated human cancer cells based solely on phenotypic single cell measurements [1].

Bray et al. reveal phenotypic profiling methods based on microscopic images of cell components or organelles to identify biologically relevant similarities and differences between samples based on these profiles and evaluate the effects of chemical, genetic or disease disorders on the phenotypic signature [2].

Collinet et al. reveal phenotypic profiling based on multi-parametric image analysis using automated confocal microscopy to analyse the human genome in terms of endocytosis of transferrin and epidermal growth factor [3].

However, methods based only on single-point readout (e.g. blood-based biomarker), such as microscopic phenotypic evaluation, are much more susceptible to failure. Therefore, the present inventors have discovered a phenotypic signature which allows remedying all the above-mentioned shortcomings in the diagnosis of inflammatory diseases, stratification of patients, monitoring the progression of diseases, and de-risking of compounds in a reliable, convenient, reproducible and cost-effective way. The results obtained by methods according to the present invention are provided by using machine learning multivariate classification algorithms that replace complex, time, and cost-intensive analysis of conventional diagnostic assays. The prior art does not disclose or propose comparable assays.

### BRIEF DESCRIPTION OF THE INVENTION

The present invention provides *in vitro* methods for diagnostic applications to identify patients suffering from inflammation-related diseases, to stratify patients, to monitor the progression of inflammation-related diseases during treatment of patients, and to identify compounds in the context of drug-de-risking to evaluate their influence on innate immune response based on the phenotypic signature, wherein the innate immune response group of each individual sample is determined by using an advanced hierarchical cluster analysis under untreated and stimulated conditions, whereby -omics data and clinical data can optionally be integrated into the phenotypic signature. The inventive phenotypic signature at the single-cell level allows conclusion on the inflammatory state of a cell obtained from a mammal based on the inflammasome activation status. The multivariate data set (data obtained by methods according to the present invention, -omics data and clinical data) representing the numerical vector that reflects the inventive phenotypic signature of the cell provides a much more robust representation of a mammal's innate immune response since measurement errors are distributed on multiple readouts compared to above mentioned conventional assays based on single-point readout.

In a first aspect thereof, the invention solves the above problem by providing an *in vitro* method for assigning a phenotypic signature of cells in a liquid biological sample obtained from a mammal, to an innate immune response group said method comprising: Stimulating said cells in said biological sample comprising cells with an inducing or repressing agent of the innate immune response; Separating said cells as stimulated from at least part of the supernatant of said biological sample; Collecting at least part of said supernatant, and analysing said supernatant for factors that induce or repress the innate immunity as released from said cells; Suitably staining of microscopically evaluable intracellular and/or extracellular structures of said stimulated cells as described herein, and subsequent microscopic evaluation of said stained intracellular and/or extracellular structures; Analysing both of said factors as released into the supernatant and said microscopic evaluation in order to obtain a combined phenotypic signature indicating the innate immune response group of said cells; and Assigning said phenotypic signature to an innate immune response group comprising the use of multivariate classification algorithms.

In a second aspect, the invention relates to an *in vitro* method for identifying whether a mammal suffers from an inflammation-related disease or is at risk of suffering from an inflammation-related disease, said method comprising: Providing a liquid biological sample obtained from said mammal comprising cells; Stimulating said cells in said biological sample with an inducing or repressing agent of the innate immune response; Separating said cells as stimulated from at least part of the supernatant of said biological sample; Collecting at least part of said supernatant, and analysing said supernatant for factors that induce or repress the innate immunity as released from said cells; Suitably staining of microscopically evaluable intracellular and/or extracellular structures of said stimulated cells as described herein, and subsequent microscopic evaluation of said stained intracellular and/or extracellular structures; Analysing both of said factors as released into the supernatant and said microscopic evaluation in order to obtain a combined phenotypic signature indicating the innate immune response group of said cells, and; Assigning said phenotypic signature to an innate immune response group comprising the use of multivariate classification algorithms, wherein a change of said phenotypic signature of said mammal when compared to said phenotypic signature in a healthy mammal identifies a mammal suffering from an inflammation-related disease or being at risk of suffering from an inflammation-related disease.

In a third aspect, the invention relates to an *in vitro* method for stratifying a mammal suffering from an inflammation-related disease, said method comprising: Providing a liquid biological sample obtained from said mammal comprising cells; Stimulating said cells in said biological sample with an inducing or repressing agent of the innate immune response; Separating said cells as stimulated from at least part of the supernatant of said biological sample; Collecting at least part of said supernatant, and analysing said supernatant for factors that induce or repress the innate immunity as released from said cells; Suitably staining of microscopically evaluable intracellular and/or extracellular structures of said stimulated cells as described herein, and subsequent microscopic evaluation of said stained intracellular and/or extracellular structures; Analysing both of said factors as released into the supernatant and said microscopic evaluation in order to obtain a combined phenotypic signature indicating the innate immune response group of said cells, and; Assigning said phenotypic signature to an innate immune response group comprising the use of multivariate classification algorithms; wherein said mammal is stratified for a treatment against said inflammation-related disease, if the phenotypic signature of said mammal deviates from the same phenotypic signature in a healthy mammal.

In a fourth aspect, the invention relates to an *in vitro* method for monitoring the progression of an inflammation-related disease in a mammal being treated against an inflammation-related disease or being at risk of suffering from an inflammation-related disease, comprising: Providing a liquid biological sample obtained from said mammal comprising cells; Stimulating said cells in said biological sample with an inducing or repressing agent of the innate immune response; Separating said cells as stimulated from at least part of the supernatant of said biological sample; Collecting at least part of said supernatant, and analysing said supernatant for factors that induce or repress the innate immunity as released from said cells; Suitably staining of microscopically evaluable intracellular and/or extracellular structures of said stimulated cells as described herein, and subsequent microscopic evaluation of said stained intracellular and/or extracellular structures; Analysing both of said factors as released into the supernatant and said microscopic evaluation in order to obtain a combined phenotypic signature indicating the innate immune response group of said cells, and; Assigning said phenotypic signature to an innate immune response group comprising the use of multivariate classification algorithms; wherein, during said treatment of said mammal, the influence of said treatment on the progression of the inflammation-related disease is monitored by a change in said phenotypic signature of said mammal.

In a fifth aspect, the invention relates to an *in vitro* method for identifying a compound and/or environmental condition that induces or represses the innate immune response of cells in a liquid biological sample obtained from a mammal, said method comprises: Contacting said cells in said liquid biological sample with a compound and/or exposing said cells in said liquid biological sample to environmental conditions that potentially induce or repress the innate immune response of said cells; Separating said cells from at least part of the supernatant of said liquid biological sample; Collecting at least part of said supernatant, and analysing said supernatant for factors released from said cells that induce or repress the innate immunity; Suitably staining of microscopically evaluable intracellular and/or extracellular structures of said contacted cells, and subsequent microscopic evaluation of said stained intracellular and/or extracellular structures; Analysing of both said factors as released into the supernatant and said microscopic evaluation in order to obtain a combined phenotypic signature indicating the innate immune response group of said cells, Identifying said compound as inducing or repressing said innate immune response based on said phenotypic signature as obtained, using multivariate classification algorithms.

In a sixth aspect, the invention relates to a phenotypic signature obtained from the methods according to the present invention.

In a seventh aspect, the invention relates to a use of a phenotypic signature as described herein for determining the innate immune response group of a mammalian cell, or for identifying whether a mammal suffers from an inflammation-related disease or is at risk of suffering from an inflammation-related disease, or for stratifying a mammal suffering from an inflammation-related disease, or for monitoring the progression of an inflammation-related disease in a mammal, or for identifying a compound and/or environmental condition that induces or represses the innate immune response of mammalian cells.

### DETAILED DESCRIPTION OF THE INVENTION

In the following, the features of the invention will be described further. These features are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

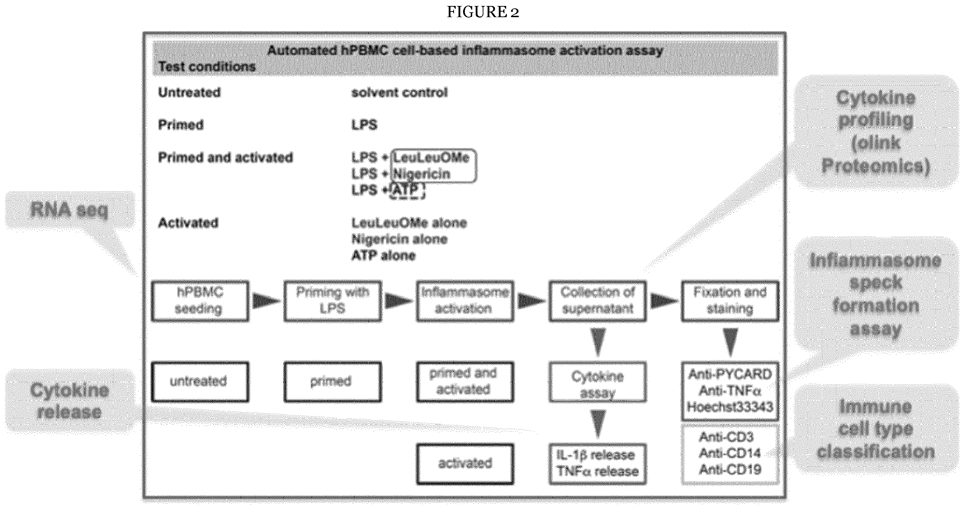

As mentioned above, in the first aspect thereof, the present invention relates to an in vitro method for assigning a phenotypic signature of cells in a liquid biological sample obtained from a mammal, to an innate immune response group said method comprising: Stimulating said cells in said biological sample comprising cells with an inducing or repressing agent of the innate immune response; Separating said cells as stimulated from at least part of the supernatant of said biological sample; Collecting at least part of said supernatant, and analysing said supernatant for factors that induce or repress the innate immunity as released from said cells; Suitably staining of microscopically evaluable intracellular and/or extracellular structures of said stimulated cells as described herein, and subsequent microscopic evaluation of said stained intracellular and/or extracellular structures; Analysing both of said factors as released into the supernatant and said microscopic evaluation in order to obtain a combined phenotypic signature indicating the innate immune response group of said cells; and Assigning said phenotypic signature to an innate immune response group comprising the use of multivariate classification algorithms. Figure 2 schematically illustrates the assay workflow.

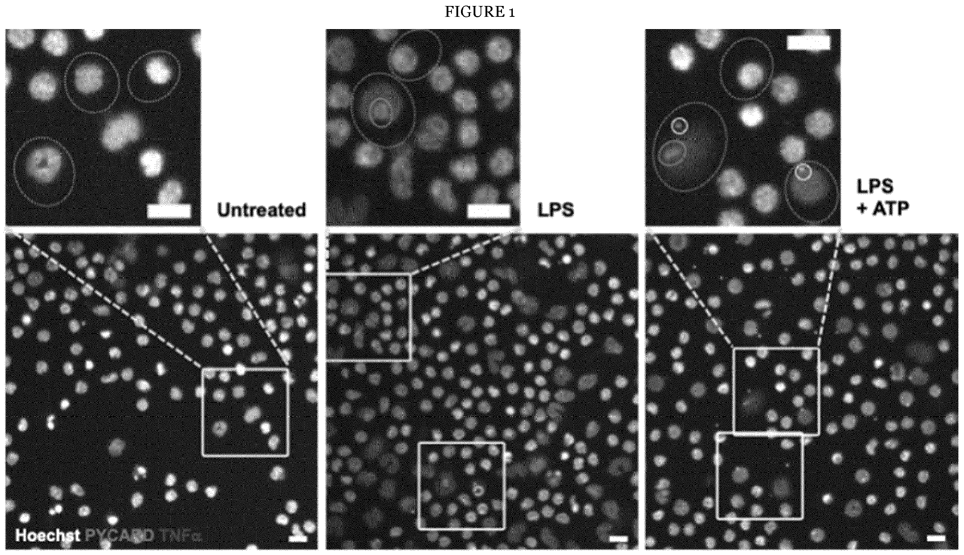

Stimulation of cells in a liquid biological sample may also covers previous priming with activators of the TLR2/4 receptor, such as for example, lipopolysaccaride (LPS) and subsequent stimulation of said primed cells with an inducing or repressing agent of the innate immune response, such as, for example, pathogen-associated molecular patterns (PAMPs) or Danger-associated molecular patterns (DAMPs). Priming of said cells induce the activation of specific cellular pathways and trigger the expression of a number of genes involved in the immune response (e.g. TNF-a). The triggered cells in said biological sample are ready to initiate an inflammatory response when stimulated with an inducing or repressing agent of the innate immune response with focus on but not limited to the toll like receptors (TLRs) and the nucleotide-binding oligomerization domain-like receptors, or NOD-like receptors (NLRs). Figure 1 shows microscopic images of representative phenotypes after staining certain cellular structures (chromatin, PYCARD and TNF-alpha) of untreated peripheral blood mononuclear cells (PBMCs), PBMCs primed with LPS and PBMCs previously primed with LPS and subsequently stimulated with adenosine triphosphate (ATP), an inducing agent of the innate immune response.

As used herein, the term "phenotypic signature" covers a multivariate data set of single cells of interest in form of a numerical vector obtained by the methods according to the present invention under different functional stimuli for the innate immune system with focus on but not limited to the toll like receptors (TLRs) and the nucleotide-binding oligomerization domain-like receptors, or NOD-like receptors (NLRs), as described herein, wherein these quantitative data may be combined with other information, such as, for example, clinical data, and/or anamnesis data. The readout core is based on the integration of said different readouts including but not limited to readout of cellular phenotypic descriptors obtained by microscopic evaluation, biochemical readout including but not limited to cytokine analysis and relevant proteins, genetic readout including but not limited to transcriptomic and genomic analysis, proteomic and clinical data. A phenotypic signature consists at least of the data from the analysis of released factors in the supernatant (TNF-alpha, and IL-1-beta) and data from the microscopic evaluation (chromatin, PYCARD, TNF-alpha and CD staining).

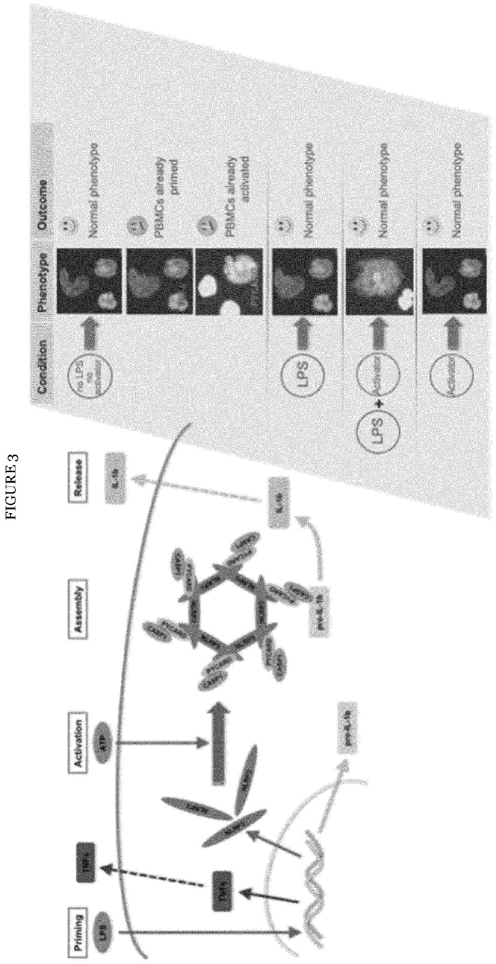

A machine learning multivariate classification algorithm processes the data set obtained by the methods according to the present invention and generates therefrom the numerical vector representing the phenotypic signature indicating the innate immune response of said cell from said mammal or of cells from control samples. The baseline signature of such control samples is composed by data collection of control cells from samples obtained from healthy donors. This data set also represents the baseline to which all the cells in the samples tested will be referenced to. Once all data has been collected the data points of the vector undergo a statistical quality check and the most robust data points are used in the final analysis. All numerical vectors of analyzed cells are then classified using said machine learning multivariate classification algorithms to finally assign the sample to an innate immune response group (i.e. healthy or diseased). Hence, each sample is represented by one numeric vector. Figure 3 exemplarily shows this assignment of untreated, or primed, or primed and activated PBMCs to their respective phenotype.

As used herein, the term "multivariate classification algorithms" means the process of identifying similarities between the multivariate data set representing the phenotypic signature of said cells as obtained by the methods according to the present invention and the multivariate data sets representing phenotypic signatures from control cells, phenotypic signatures from untreated cells with known innate immune response, or phenotypic signatures from reference cells with known innate immune response contained in a database. Conventional multivariate classification algorithms are available for the skilled person and may be adapted for the purpose of the present invention. For example, Nicholson et al. reveals machine learning multivariate pattern analysis for classification of posttraumatic stress disorder. Apart from that, there is a variety of further documents disclosed in the prior art (Nicholson, Densmore, Kinnon, Cambridge University Press; Volume 49, Issue 12; September 2019, pp. 2049-2059: Machine learning multivariate pattern analysis predicts classification of posttraumatic stress disorder and its dissociative subtype: a multimodal neuroimaging approach).

As described above, for assigning a numerical vector of a cell of interest to an innate immune response group the data points of said numerical vector are compared with data points of numerical vectors present in a database that stores a number of data representing "innate immune response groups". The term used herein, covers the cluster in which phenotypic signatures are assigned by said multivariate algorithm due to similarities in their phenotypic signatures. For example, a cluster includes numerical vectors of healthy donors, since the phenotypic signature of healthy donors have similarities in their data sets identified by the multivariate classification algorithm. In addition to the data of the "healthy" immune status group, the database contains further data sets of sample controls, e.g. from donors with inflammation-related diseases. These samples (from healthy and diseased donors) form a reference data set or baseline. Using the multivariate classification algorithm for machine learning, each individual sample of a cell from a mammal of interest is analyzed and assigned to the respective innate immune response group (e.g. healthy, or diseased innate immune response group).

As used herein, the term "innate immune response" covers any reaction, interaction or signalling pathway associated with the innate immune system. The innate immune response also covers the innate immune status of a cell at any given time, which describes the static innate immune status of a cell at a given time with a focus on activated toll-like receptors (TLRs) and NOD-like receptors (NLRs) and the NLRP3, AIM2, and NLRC4 inflammasome activation status in addition to the immune cells usually analysed. The term "innate immune response group" describes a group of cells, wherein all cells in said respective group have a consistent and/or similar innate immune response and/or activation state of their innate immune status.

As used herein the term "liquid biological sample" means any mammalian body fluid containing the cells to be analyzed from said mammal, which enables the methods to be carried out in accordance with the present invention. For example, such liquid biological samples may be selected from blood, serum, and saliva, and a tissue, organ or cell type blood sample but without being limited to it.

The term "in vitro" as used herein, refers to methods that are carried out externally to a mammal and in a controlled artificial environment. For example, the cells for an "in vitro" method may be taken from said mammal, or may be obtained from immortal cell lines. These methods may be performed, for example, in a test tube or Petri dish, but without being limited to these.

The term "supernatant" as used herein means the nutrient medium required for the cultivation and storage of said cells of interest. The culture supernatant is in direct contact with said cells and thus contains released factors by said cells which are essential for obtaining a phenotypic signature of said cells.

In the second aspect, the invention relates to an *in vitro* method for identifying whether a mammal suffers from an inflammation-related disease or is at risk of suffering from an inflammation-related disease, said method comprising: Providing a liquid biological sample obtained from said mammal comprising cells; Stimulating said cells in said biological sample with an inducing or repressing agent of the innate immune response; Separating said cells as stimulated from at least part of the supernatant of said biological sample; Collecting at least part of said supernatant, and analysing said supernatant for factors that induce or repress the innate immunity as released from said cells; Suitably staining of microscopically evaluable intracellular and/or extracellular structures of said stimulated cells as described herein, and subsequent microscopic evaluation of said stained intracellular and/or extracellular structures; Analysing both of said factors as released into the supernatant and said microscopic evaluation in order to obtain a combined phenotypic signature indicating the innate immune response group of said cells, and; Assigning said phenotypic signature to an innate immune response group comprising the use of multivariate classification algorithms, wherein a change of said phenotypic signature of said mammal when compared to said phenotypic signature in a healthy mammal identifies a mammal suffering from an inflammation-related disease or being at risk of suffering from an inflammation-related disease.

In the third aspect, the invention relates to an *in vitro* method for stratifying a mammal suffering from an inflammation-related disease, said method comprising: Providing a liquid biological sample obtained from said mammal comprising cells; Stimulating said cells in said biological sample with an inducing or repressing agent of the innate immune response; Separating said cells as stimulated from at least part of the supernatant of said biological sample; Collecting at least part of said supernatant, and analysing said supernatant for factors that induce or repress the innate immunity as released from said cells; Suitably staining of microscopically evaluable intracellular and/or extracellular structures of said stimulated cells as described herein, and subsequent microscopic evaluation of said stained intracellular and/or extracellular structures; Analysing both of said factors as released into the supernatant and said microscopic evaluation in order to obtain a combined phenotypic signature indicating the innate immune response group of said cells, and; Assigning said phenotypic signature to an innate immune response group comprising the use of multivariate classification algorithms; wherein said mammal is stratified for a treatment against said inflammation-related disease, if the phenotypic signature of said mammal deviates from the same phenotypic signature in a healthy mammal.

In the fourth aspect, the invention relates to an *in vitro* method for monitoring the progression of an inflammation-related disease in a mammal being treated against an inflammation-related disease or being at risk of suffering from an inflammation-related disease, comprising: Providing a liquid biological sample obtained from said mammal comprising cells; Stimulating said cells in said biological sample with an inducing or repressing agent of the innate immune response; Separating said cells as stimulated from at least part of the supernatant of said biological sample; Collecting at least part of said supernatant, and analysing said supernatant for factors that induce or repress the innate immunity as released from said cells; Suitably staining of microscopically evaluable intracellular and/or extracellular structures of said stimulated cells as described herein, and subsequent microscopic evaluation of said stained intracellular and/or extracellular structures; Analysing both of said factors as released into the supernatant and said microscopic evaluation in order to obtain a combined phenotypic signature indicating the innate immune response group of said cells, and; Assigning said phenotypic signature to an innate immune response group comprising the use of multivariate classification algorithms; wherein, during said treatment of said mammal, the influence of said treatment on the progression of the inflammation-related disease is monitored by a change in said phenotypic signature of said mammal.

"Monitoring" as the term is used in the context of the present invention means the regular provision of said liquid biological sample from said mammal, as described herein, and the performance of the methods according to the present invention to provide a reliable and complete indication of the progression of a mammalian disease during treatment. The intervals can be determined by a skilled person.

Preferred is an embodiment of the method according to the present invention, wherein said inducing or repressing agent of the innate immune response induces or represses the activity of a factor selected from the group consisting of Toll-like receptors (TLRs) and/or Nod-like receptors (NLRs), such as, for example TLR4/TLR2, NLPR3, AIM2 and/or NLRC4, and is preferably selected from lipopolysaccharide (LPS), LeuLeuOMe, Nigericin, ATP, MSU crystals, aluminium crystals, nano-SiO2, CPPD crystals, cholesterol crystals, Poly(dA:dT), nanoparticles, flagellin, therapeutic antibodies, CAR-T cells, amyloid beta, Tau, alpha-synuclein, TDP43, exosomes, and nanoplastic, but without being limited to these.

The term "inducing or repressing agent", as used in the context of the present invention, describes a substance which affects an increase or decrease in the innate immune response, wherein an "induction" induces intracellular and/or extracellular inflammatory signalling pathways and a "repression" inhibits intracellular and/or extracellular inflammatory signalling pathways. For example, the use of an inducing agent may trigger the expression of a number of genes involved in the innate immune response, such as, for example the gene for expression of the cytokine protein ILi-beta.

As used herein the term "toll-like receptors (TLRs)" means structures of the innate immunity and belong to a group of Pattern-Recognition Receptors (PRRs). TLRs are transmembrane proteins and are used for the recognition of PAMPs (Pathogen-Associated Molecular Patterns). PAMPs are highly conserved, pathogen-specific patterns, which are not found in mammals. Such PAMPs can be, for example, proteins, lipids and carbohydrates, such as, for example, lipopolysaccharides or flagellin that occur on the surface of the microorganisms. In contrast, so called "damage-associated molecular patterns (DAMPs)" are host molecules, such as for example heat shock protein (HSPs), or Interleukin-i (IL-1). These are endogenous, normally intracellular molecules that enter the extracellular space when a cell is damaged. By binding to the PRRs, an inflammatory reaction is triggered.

The term "NOD-like receptors (NLRs)" as used herein covers structures of the innate immunity and also belong to a further group of PRRs. In contrast to TLRs, NLRs are present as soluble proteins in the cytosol and are responsible for recognition of intracellular PAMPs that were introduced into the cell via bacterial protein secretion or PAMPs that were taken up by immune cells via phagocytosis.

In the fifth aspect, the invention relates to an *in vitro* method for identifying a compound and/or environmental condition that induces or represses the innate immune response of cells in a liquid biological sample obtained from a mammal, said method comprises: Contacting said cells in said liquid biological sample with a compound and/or exposing said cells in said liquid biological sample to environmental conditions that potentially induce or repress the innate immune response of said cells; Separating said cells from at least part of the supernatant of said liquid biological sample; Collecting at least part of said supernatant, and analysing said supernatant for factors released from said cells that induce or repress the innate immunity; Suitably staining of microscopically evaluable intracellular and/or extracellular structures of said contacted cells, and subsequent microscopic evaluation of said stained intracellular and/or extracellular structures; Analysing of both said factors as released into the supernatant and said microscopic evaluation in order to obtain a combined phenotypic signature indicating the innate immune response group of said cells; Identifying said compound as inducing or repressing said innate immune response based on said phenotypic signature as obtained, using multivariate classification algorithms.

In the context of the present invention the term "environmental condition" covers all surrounding factors that are exposed to said cell that may have a potential effect of inducing or repressing the innate immune response of said cell. Such an environmental condition may be selected from overpressure, vacuum, heat, heavy metals, or ultraviolet radiation, but without being limited to these. As a result of stressful environment such a condition may lead, for example, to the production of heat shock proteins (HSPs). Such that HSPs can bind to several pathogen-associated molecular patterns (PAMPs) and enhance Toll-like receptor (TLR) ligand-induced stimulation.

Further preferred is an embodiment of the method according to the present invention, wherein said compound is selected from a drug, a small molecule, a polypeptide, an antibody or fragment thereof, a peptide, a polynucleotide, an oligonucleotide, nanoparticles, a carbohydrate, or lipids.

The term "drug" as used in the context of the present invention includes any substance or pharmaceutical composition intended to cure or prevent disease in a mammal. The drug may be an already known substance or pharmaceutical composition, but it also includes those substances or pharmaceutical compositions which are not yet known. The term "pharmaceutical composition" covers all medical preparations, wherein the substances do not occur in their pure form but in their form as an administrable pharmaceutical composition, since the pure substances are formulated in combination with excipients to form a medicinal product. For example, it may be necessary to coat the compound with, or co-administer the compound with, a material to prevent its inactivation, or the compound may be administered to a mammal in an appropriate carrier, for example, in liposomes, or a diluent. Pharmaceutically acceptable diluents include saline and aqueous buffer solutions, for example to bring the substance in form of tablets, capsules, injections, infusions, solutions, salves or syrups. In addition to the pure substances, excipients or the entire drug may also induce or repress the innate immune response, whereby this inducing or repressing impact can be identified with the methods according to the invention based on the phenotypic signature of said cell from said mammal.

The term "small molecules" as used herein comprises low molecular weight compounds with a low molecular mass. This refers to a class of active substances with a molecular mass not exceeding approximately 800 g-mol-i.

In the context of the present invention the term "protein" is used to denote a polymer composed of amino acid monomers joined by peptide bonds. It refers to a molecular chain of amino acids, and does not refer to a specific length of the product and if required can be modified in *vivo* or *in vitro,* for example by glycosylation, amidation, carboxylation or phosphorylation. Amino acid chains with a length of less than approx. 100 amino acids are called "peptides". The terms "peptides", and "proteins" are included within the definition of "polypeptides". A "peptide bond" is a covalent bond between two amino acids in which the α-amino group of one amino acid is bonded to the α-carboxyl group of the other amino acid. All amino acid or polypeptide sequences, unless otherwise designated, are written from the amino terminus (N-terminus) to the carboxy terminus (C-terminus).

The term "antibody" as referred to herein includes whole, full length antibodies and any fragment or derivative thereof in which the "antigen-binding portion" or "antigen-binding region" or single chains thereof are retained, such as a binding domain of an antibody specific for the immunogenic active moiety of the conjugate. A naturally occurring "antibody" (immunoglobulin) is a glycoprotein comprising at least two heavy (H) chains and two light (L) chains inter-connected by disulfide bonds. Each heavy chain is comprised of a heavy chain variable region (abbreviated herein as VH) and a heavy chain constant region. Heavy chains are classified as mu, delta, gamma, alpha, or epsilon, and define the antibody's isotype as IgM, IgD, IgG, IgA, and IgE, respectively. The heavy chain constant region is comprised of three domains, CHI, CH2 and CH₃. Each light chain is comprised of a light chain variable region (abbreviated herein as VL) and a light chain constant region. The light chain constant region is comprised of one domain, CL. The VH and VL regions can be further subdivided into regions of hyper variability, termed complementarity determining regions (CDRs), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, FR4. The heavy and light chains form two regions: the Fab (fragment, antigen binding) region, also referred to as the variable (Fv) region, and the Fc (fragment, crystallizable) region. The variable regions (Fv) of the heavy and light chains contain a binding domain that interacts with an antigen. The constant (Fc) regions of the antibodies may mediate the binding to host tissues or factors, including various cells of the immune system (e.g., effector cells) and the first component (Clq) of the classical complement system. The term "Fc" as used herein includes native and mutein forms of polypeptides derived from the Fc region of an antibody. Truncated forms of such polypeptides containing the hinge region that promotes dimerization also are included. Fusion proteins comprising Fc moieties (and oligomers formed therefrom) offer the advantage of facile purification by affinity chromatography over Protein A or Protein G columns. One suitable Fc polypeptide is derived from the human IgGi antibody.

As used herein the term "polynucleotide" covers linear biopolymers composed of 20 or more nucleotides with alternating nucleic bases. Nucleic acid polymers with a smaller number of nucleotides are called "oligonucleotides". Such polynucleotides may be, for example, DNA, cDNA, RNA or synthetically produced DNA or RNA or a recombinant produced chimeric nucleic acid molecule comprising one of these nucleic acids alone or in combination.

The term "nanoparticle" as used herein defines particles with a particle size smaller than 0.1 µm (< 100 nm) that are either generated naturally or can be synthetically produced. Such nanoparticles can be, for example, carbon-containing nanoparticles, metal and semi-metal oxides, semiconductors, metal sulphides or polymers, but without being limited to these.

As used herein the term "carbohydrates" covers all carbon compounds, consisting of carbon, hydrogen and oxygen, which may be present as monosaccharides or as polysaccharides.

The term "lipid" as used herein refers to the totality of fats and fat-like substances. Lipids are chemically heterogeneous substances that dissolve poorly in water, but well in nonpolar solvents.

Preferred is an embodiment of the method according to the present invention, wherein said inflammation-related disease is an autoimmune disease, or preferably a neurodegenerative disease.

The term "inflammation-related diseases", as used in the context of the present invention, comprises any disease, which consequence is the activation of the immune system of a mammal. The term "inflammation-related diseases" covers "neurodegenerative diseases" and "autoimmune diseases", but without being limited to it. As a further embodiment, the term also covers any cancer disease.

As used herein the term "autoimmune disease" covers any disease with reactions of the mammals body that are based on a disturbed tolerance of the immune system to substances of the own body. Such autoimmune disease may be selected from autoimmune diseases, such as idiopathic or secondary pulmonary fibrosis, liver fibrosis, non-alcoholic hepatitis (NASH), autoimmune hepatitis, giant cell hepatitis, primary sclerosing cholangitis, primary biliary cirrhosis, systemic lupus erythematosus, lupus nephritis, good pasture syndrome, arthritis, sarcoidosis, allergic bronchopulmonary aspergillosis, keloid disease, such as for example fibroproliferative dermal tumor, collagen vascular diseases, systemic sclerosis, viral induced autoimmune diseases, but without being limited to.

The term "neurodegenerative diseases" as used herein, means any disease that primarily affect the nerve cells in the mammals brain and lead to progressive degeneration and death of the nerve cells, such as, for example, Parkinson, prion diseases, motor neuron diseases (MND), Huntington disease (HD), spinocerebellar ataxia (SCA), spinal muscular atrophy (SMA), and most preferably Alzheimer diseases, but without being limited to.

Further preferred is an embodiment of the method according to the present invention, wherein in step of analyzing of both said factors as released into the supernatant and said microscopic evaluation in order to obtain a combined phenotypic signature a proteome, transcriptome, lipidome and/or genome of said cells is evaluated, and clinical data are included to supplement said combined phenotypic signature.

As used herein, the term "proteome", refers to the totality of proteins expressed by further single cells obtained from said mammal that are evaluated parallel to the microscopic evaluation of said cell in said liquid biological sample from said mammal according to the methods of the present invention. The expressed proteins may occur as intracellular, extracellular or transmembrane proteins. Many methods for the analysis of these proteins are known to the skilled person. For example, the proteome can be analysed with Western blot or mass spectrometry, but without being limited to these methods. The amount of cellular proteins may vary depending on the time of measurement.

The term "transcriptome" as used herein, refers to the totality of all genes transcribed in further single cells (i.e. the entirety of all RNA molecules present in said further single cells) obtained from said mammal that are evaluated parallel to the microscopic evaluation of said cell in said liquid biological sample from said mammal according to the methods of the present invention. The transcribed RNA molecules are present intracellularly. There is a number of different methods in the prior art for the analysis of cellular RNA, such as the reverse transcriptase polymerase chain reaction (RT-PCR), but without being limited to this method. The amount of RNA present in a cell can vary depending on the time of measurement.

As used herein, the term "lipidome" refers to the totality of all lipids in further single cells obtained from said mammal that are evaluated parallel to the microscopic evaluation of said cell in said liquid biological sample from said mammal according to the methods of the present invention. There are many methods known to the skilled person, such as mass spectrometry, to analyse the lipidome, but without being limited to this.

The term "genome" as used herein refers to the entirety of the genetic information in further single cells obtained from said mammal that are evaluated parallel to the microscopic evaluation of said cell in said liquid biological sample from said mammal according to the methods of the present invention. The term "genetic information" covers all genetic material contained in the cell nucleus, the mitochondria and the plastids. The prior art provides a number of methods, such as, for example fluorescence in situ hybridisation (FISH) or polymerase chain reaction (PCR), but without being limited to these.

Further preferred is an embodiment of the method according to the present invention, wherein said cells are selected from immune cells, peripheral blood mononuclear cells (PBMCs), PBMCs co-cultured with other cells in 2D and 3D culture, lymphocytes and monocytes.

The term "peripheral blood mononuclear cells (PBMCs)" as used herein covers mononuclear cells of the blood, which have a round cell nucleus. After providing said liquid biological sample from said mammal, PBMCs from blood, for example, may be purified by centrifugation through a hydrophilic colloid. In the methods according to the present invention, both dead and living cells may be used.

As used herein, the term "2D culture" includes conventional cell culture under in vitro conditions, wherein adherent cells spread out on a plastic or glass substrate as a so-called "monolayer" (i.e. a single-cell layer). The may grow into a dense cell layer, but stop dividing as soon as they become too confluent. In 2D culture, cells do not continue to grow on top of each other to form a multilayer.

The term "3D culture" as used herein, refers to the cultivation of said cells in a micro-structured three-dimensional cell culture under in vitro conditions. For example, a suitable matrix can be selected from hydrogels made of scaffold proteins such as collagen, gelatine methacrylate.

As used in the context of the present invention, the term "co-cultured" describes the simultaneous in vitro cultivation of said different cell types in an artificial environment. For example, PBMCs from said liquid biological sample from said mammal may be embedded in 2D culture or 3D culture and cultivated simultaneously. The skilled person is aware of the required knowledge.

Further provided is an embodiment of the method according to the present invention, wherein said mammal is selected from a mouse, a rat, a monkey, a rabbit, a pig, a donkey, a cow, a horse, a sheep, and preferably a human.

Yet preferred is an embodiment of the method according to the present invention, wherein said separating comprises immobilizing said cells on a solid carrier material that is transparent, preferably selected from glass or a plastic material, and preferably chemically inert.

The term "solid carrier" refers to any carrier which is insoluble and chemically inert under the reaction conditions without having an influence on cellular reaction. Furthermore, the carrier must allow the substrate to flow in and out. A number of suitable carriers are known for cell immobilization.

As used herein, the term "immobilizing" refers to the spatial fixation of said cell in the liquid biological sample from said mammal to said solid carrier, for example by the action of chemicals, such as, for example, methanol, ethanol, or acetone, but without being limited to these.

In a further preferred embodiment of the method according to the present invention said microscopically evaluable intracellular and/or extracellular structures of said cells to be stained comprise lipids, sugars, nucleic acids, functional compartments and/or intracellular and/or extracellular proteins, such as for example, PYCARD, TNF-alpha, CD14, CD19, CD3, IL-1-beta, CASP1, NFkappaB, and NLRP3. The microscopic evaluation is performed optically. The skilled person is familiar with a variety of methods that can be used for the optical detection of cellular structures. For example, neutral dyes are bound by the lipid structures, and alkaline dyes may be used for staining of acidic nucleic acids, but without being limited to these. For evaluation of intracellular and/or extracellular proteins, such as for example, PYCARD, TNF-alpha, CD14, CD19, CD3, IL-1-beta, CASP1, NFkappaB, and NLRP3 conventional immunofluorescence may be applied, using commercially available antibodies that bind specific to the respective proteins. By adding respective second fluorochrome-labelled detection antibodies, the corresponding structures on said proteins may be detected with a fluorescence microscope according to the wavelength range of the respective used fluorochrome.

Yet preferred is an embodiment of the method according to the present invention, wherein said released factors in the supernatant to be analysed comprise cytokines, such as for example, TNF-alpha, and IL-1-beta. As used herein, the term "released factors" covers all signal molecules that may be secreted or released by said living or death cells into the surrounding supernatant, for example hormones, neurotransmitters, nitric oxide and especially cytokines, but without being limited to these. The term "cytokines", as used in the context of the present invention, covers all proteins secreted by said cells in the course of an immunological or inflammatory reaction. The skilled person knows a number of methods for analysis of released factors, in particular cytokines, such as, for example, enzyme-linked immune absorbent spot (ELISpot), or enzyme-linked immunosorbent assay (ELISA), but without being limited to these.

In yet another preferred embodiment of the method according to the present invention, said microscopic evaluation comprises at least one of morphology, subcellular location of said stained intracellular structures, and marker intensity of said stained intracellular structures of said cells.

The term "morphology" as used in the context of the present invention describes the shape and structure of said cells to be analysed above the molecular level.

As used herein, the term "subcellular location" means the intracellular position of the stained intracellular structures within said cells or extracellular structures on the surface of the cells.

The term "marker intensity" as used herein, describes the strength in which the detection signal is emitted. The strength of the detection signal may be proportional to the amount of detectable intracellular structures present in said cells.

Preferred is an embodiment of the method according to the present invention, wherein said evaluation of the phenotypic signature is carried out automatically.

The term "automatically" as used herein, refers to autonomous microscopic evaluation by using automated microscopy, as well as autonomous collection, storage, processing and evaluation of further data, as obtained by the methods according to the present invention, by said self-learning multivariate algorithms.

Further preferred is an embodiment of the method according to the present invention, wherein the method comprises a comparison with a control from a database.

The term "comparison" as used herein, means that the numerical vector representing the phenotypic signature as obtained by the methods according to the present invention is searched for overlap with numerical vectors representing the phenotypic signatures of controls stored in said database.

As used herein, the term "controls" covers numerical vectors of phenotypic signatures of untreated control cells obtained from healthy mammals indicating the innate immune response of healthy mammals stored in a database for comparison. The controls enable matching of the numerical vector of the phenotypic signature of said cells of interest with numerical vectors of phenotypic signatures of controls whose immune status or immune response group is known to be healthy.

The term "database" as used herein, covers a proprietary database containing phenotypic signatures of mammalian cells contacted with over 20,000 small molecules and which can be used as references. The database consists of a collection of numerical vectors representing phenotypic signatures from control cells, phenotypic signatures from untreated cells with known innate immune response or phenotypic signatures from reference cells with known innate immune response. The term "data set" as used herein covers several hundred of features.

Further preferred is an embodiment of the method according to the present invention wherein said innate immune response is determined based on changes in the phenotypic signature when compared to a control provided by a database.

"Changes" as the term is used herein, means the alteration or deviation of said phenotypic signature from a phenotypic signatures from control cells, phenotypic signatures from untreated cells with known innate immune response or phenotypic signatures from reference cells with known innate immune response, as provided by a database, to determine the innate immune response, in particular the inflammasome activation status, of a cell. Each tested biological sample from said mammal is compared to the baseline controls and the multivariate classification algorithm determines the change and assigns the numeric vector, and thus the corresponding sample, to a designated innate immune response group.

Preferred is an embodiment of the method according to the present invention, wherein said analysis of said innate immune response comprises the analysis of the inflammasome activation status of said cells in said liquid biological sample.

A further embodiment, relates to a method for obtaining a phenotypic signature to determine the innate immune status of cells from a liquid biological sample from a mammal, the method comprising: Collecting data from a database of the analysed factors as released into the supernatant of stimulated or contacted cells; Collecting data from a database of said microscopic evaluation of intracellular and/or extracellular structures of said stimulated or contacted cells; Optionally, collecting data from proteome, transcriptome, lipidome and/or genome analysis of said cells and clinical data from a database; Merging of all data sets to determine the phenotypic signature.

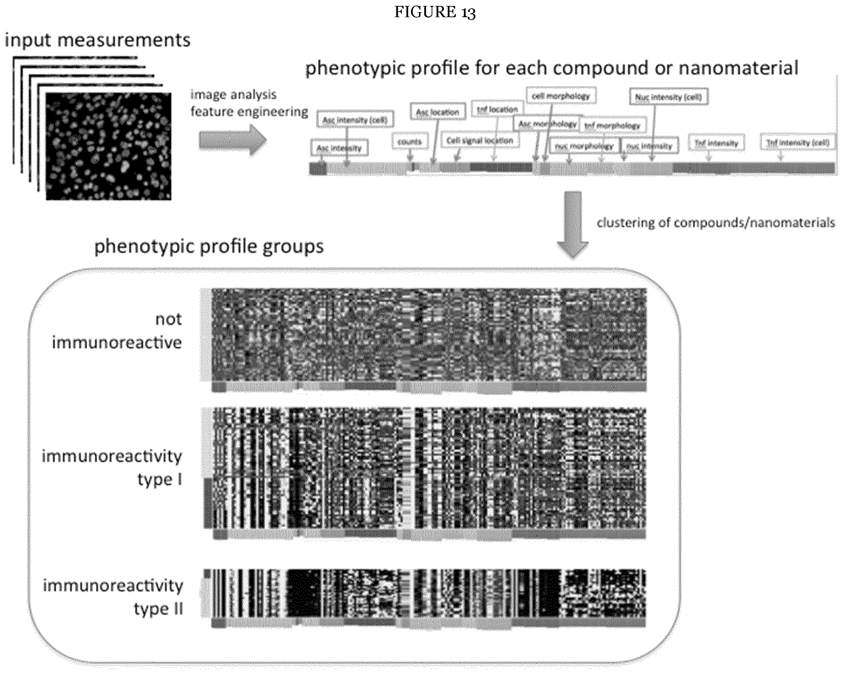

In the sixth aspect, the invention relates to a phenotypic signature obtained from the methods according to the present invention. As described herein, the phenotypic signature is composed by different sources of information. Figure 13 shows an example of such a phenotypic signature, wherein additional information may be added according to the needs in a customized manner.

In the seventh aspect, the invention relates to a use of a phenotypic signature as described herein for determining the innate immune response group of a mammalian cell, or for identifying whether a mammal suffers from an inflammation-related disease or is at risk of suffering from an inflammation-related disease, or for stratifying a mammal suffering from an inflammation-related disease, or for monitoring the progression of an inflammation-related disease in a mammal, or for identifying a compound and/or environmental condition that induces or represses the innate immune response of mammalian cells.

The terms "of the [present] invention", "in accordance with the invention", "according to the invention" and the like, as used herein are intended to refer to all aspects and embodiments of the invention described and/or claimed herein.

In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

All references, patents, and publications cited herein are hereby incorporated by reference in their entirety.

### BRIEF DESCRIPTION OF THE FIGURES

The figures show:
**Figure 1****:** shows images of representative phenotypes of PBMC under different stimuli (i.e. LPS and LPS+ATP).
**Figure 2****:** shows the schematic representation of the assay workflow from cell seeding to the cell fixation passing through the different phases of priming, activating and detection of the assay markers. At this stage different experimental quantitative information can be collected for different markers described (but not limited) to the one showed in the balloons.
**Figure 3****:** shows schematic examples of expected and altered phenotype under experimental conditions

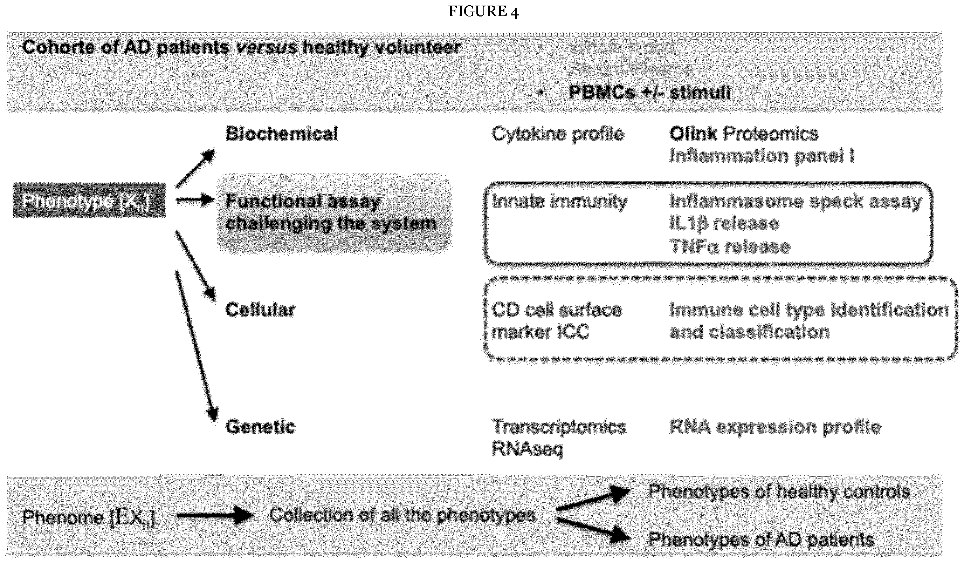

**Figure 4****:** shows the numerical vector describing the phenotypic signature, which is composed by different sources of information.
**Figure 5****:** shows the structure of the pipetting robot worktable for automated performance of the assay.
**Figure 6****:** shows the pipetting scheme for Leu Leu source plate
**Figure 7****:** shows the pipetting scheme for Nigericin source plate
**Figure 8****:** shows the assay test plate for incubation with leucine.
**Figure 9****:** shows the assay test plate for incubation with nigericin.

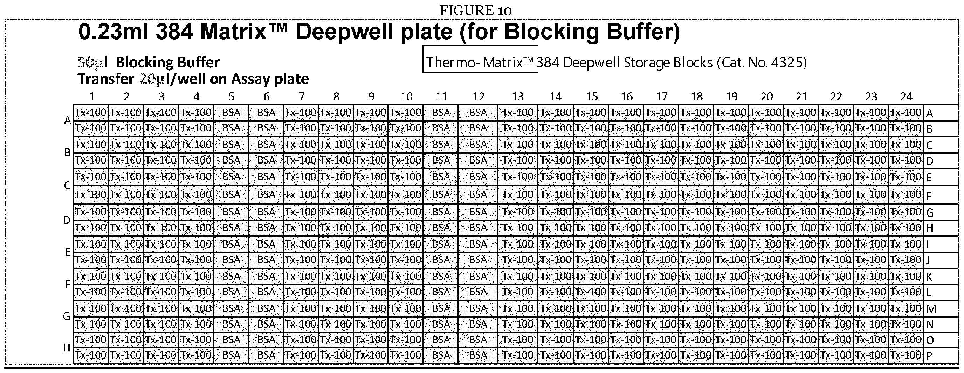

**Figure 10****:** shows the pipetting scheme for the blocking solutions.

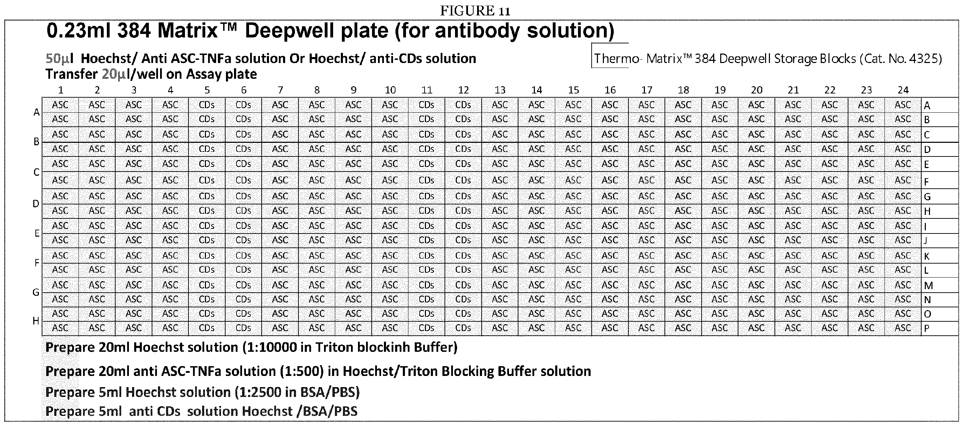

**Figure 11****:** shows the pipetting scheme for the staining solutions.

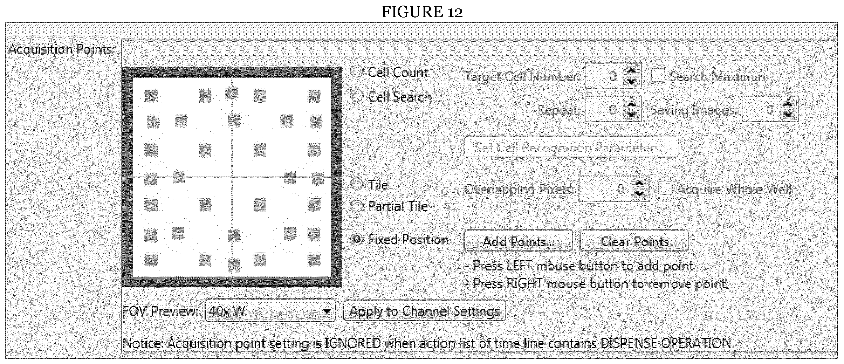

**Figure 12****:** shows the imaging of 32 fields for each well.
**Figure 13****:** shows the data analysis pipeline.
**Figure 14****:** shows de-risking Strategy. Phenotypic signatures of compounds under test are clustered (type A, B and C) with known reference compounds/conditions (a). According to the phenotypes produced and their cluster assignments, compounds under test can be risk-assessed.
**Figure 15****:** shows the summary of de-risking pain-killers (cyclooxygenase (COX)-Modulators) approach by using the test system of the present invention.

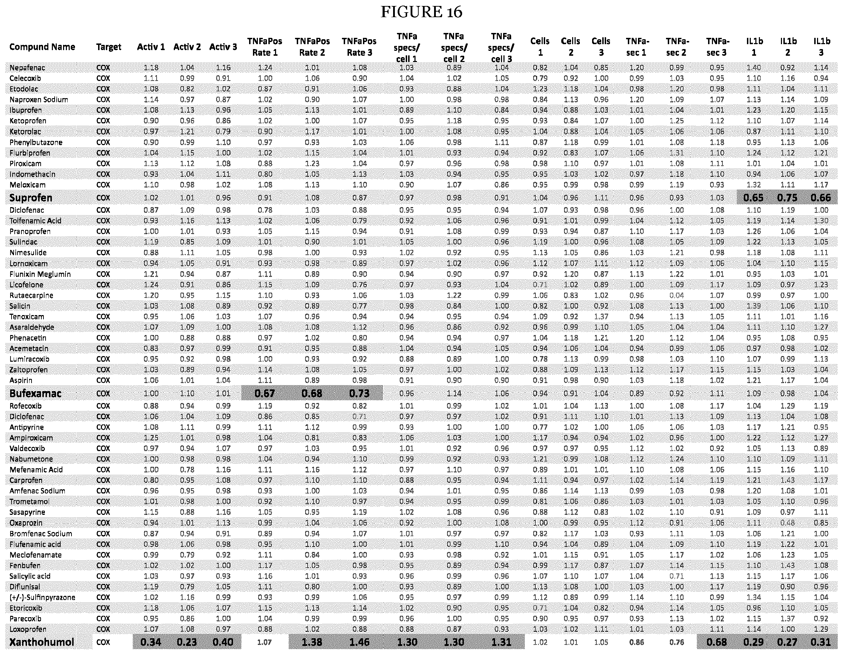

**Figure 16****:** shows the results of de-risking pain-killers (cyclooxygenase (COX)-Modulators) approach by using the test system of the present invention. 54 pain-killers were tested in three independent experiments. Six representative parameters demonstrate the unique collective phenotypic signature. The results were normalized to the untreated control group. Image-based assay read outs: a)"Active", ratio of cells presenting mounted inflammasome, b) "TNFaPosRate", ratio of cells showing intracellular TNFalpha expression, c) "TNFa specs/cell", number of TNFalpha spots within TNFalpha positive cells, d) "Cells", total number of cells. Homogenous assay read outs: e) "TNFa sec", TNFalpha concentration in the supernatant in ng/ml, f) "ILib", ILibeta concentration in the supernatant in ng/ml.
**Figure 17****:** shows Unsupervised Clustering; Screen of a compound collection processed by our unique image and data analysis pipeline. A. We identified compounds having no effect on the monitored mechanism of innate immune response and cellular status of the heterotypical cell model. B. We classified compounds, which show no phenotypic alteration compared to the untreated control (full rescue). C. We disclosed novel lead structures inducing the same cellular response as our benchmark control CRID3/MCC950 (drug in development). D. Compounds, which induce new cellular phenotypes.
**Figure 18****:** shows compound-induced phenotypic rescue; The immunX approach identifies compounds, which show complete inhibition of the innate immune response in our cell model and completely rescue the "activation phenotype" towards the "no activation phenotype".

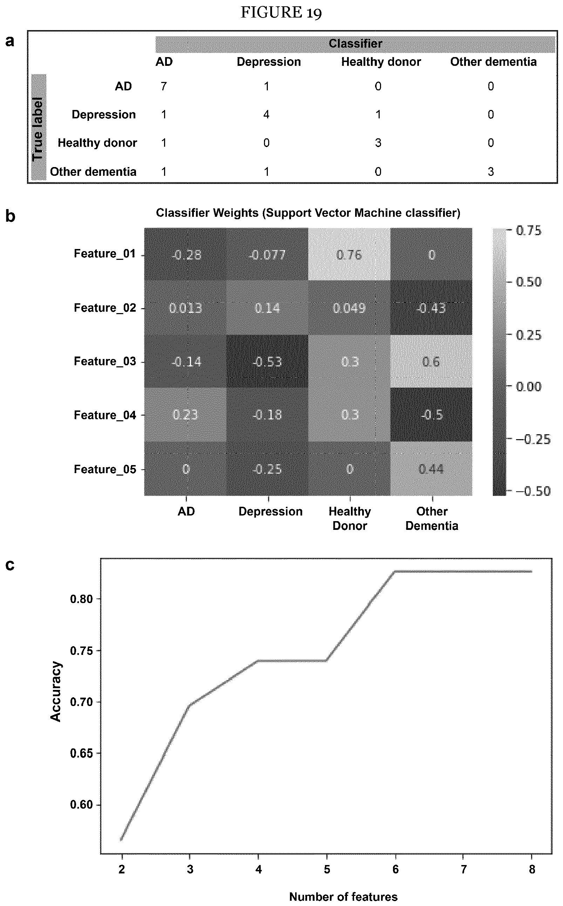

**Figure 19****:** Classification of the disease status via a Support Vector Machine (SVM) classifier and based on a 72 features derived from a multivariate dataset. (a) Confusion matrix to evaluate the quality of the output of the classifier allowing 5 features to be selected for classification. The diagonal elements represent the number of patient samples for which the predicted label is equal to the true label, while off-diagonal elements are those that are mislabeled by the classifier.(b) Classifier weights of the selected features used by the SVM classifier for the class assignment of the patient samples. (c) Accuracy of the classification according to the number of features used for the classifier.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

### Example 1:

### PBMC-Assay to identify whether a subject suffers from an inflammation-related disease

PBMC samples from 5 patients and 3 healthy donors, as controls, were provided. Subsequently, reagents, the PBMC cell suspensions in complete medium (RPMI 1640 (Biochrom Cat. No. F1215 Lot 1106E), 4 mM L-Alanyl-Glutamine (Biochrom, Cat. No. K 302, 200mM), 25 mM HEPES (LifeTechnologies, Cat. No. 15630-56, 1M), 1 mg/ml Ciprofloxacin (Kabi, Infusionslsg., 200mg/ml), 10% Hi-FBS (Sigma, Cat. No. F9665, Charge 065M3367)) and a lipopolysaccharide (LPS-EB Ultrapure Lipopolysaccharide from E. coli 0111:B4 strain- TLR4 ligand, 5 x 106 EU; InvivoGen, Cat.No. tlrl-3pelps; LOT number: L3P- L3P-38-02; 5 x 10⁶ EU of ultrapure LPS) stock solution in endotoxin-free water with a concentration of 5mg/ml (corresponds to 5 x 106 EU/ml) were prepared. From this, an LPS solution 1 in complete medium with a concentration of 500 ng/ml was prepared. Subsequently, a LPS solution 2 in complete medium with a concentration of 60ng/ml was prepared. 50 µl/well of complete medium and LPS solutions 2 were pipetted in a 384 Deepwell (DW)-plate according to the pipetting scheme shown in Table 1 below.

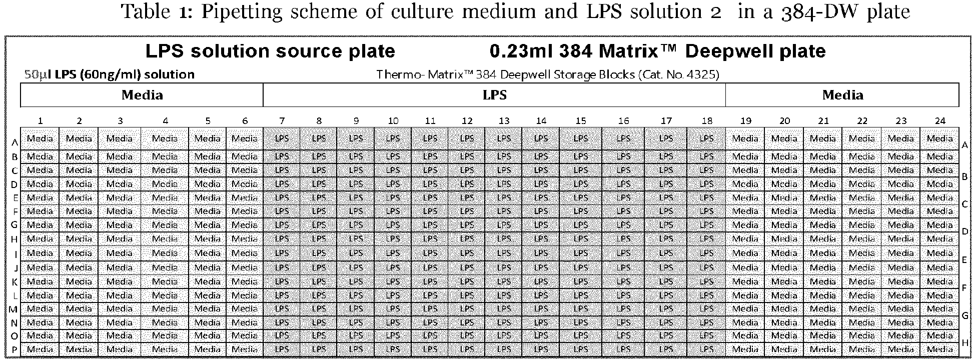

The plate together with medium and LPS solution 2 was stored at 37°C until priming of the PBMC cells.

4.7 ml of cell suspension (4.5 ml for cell seeding into the 384-DW plate and 0.2 ml for subsequent RNA sequencing) of each individual PBMC sample from each donor were prepared in a vial so that the respective cell suspension had a final concentration of 1.0 x 106 cells/ml.

RNA-Sequencing sample preparation: To prepare the samples for RNA sequencing, 2.0 x 10⁵ cells were transferred from the respective cell suspension of each PBMC sample into a separate tube. The samples were sent to an external laboratory for RNA sequencing.

340 µl of the provided cell suspensions from the five patients and three healthy donors were pipetted per well into a 96 DW -plate according to the pipetting scheme shown in Table 2.

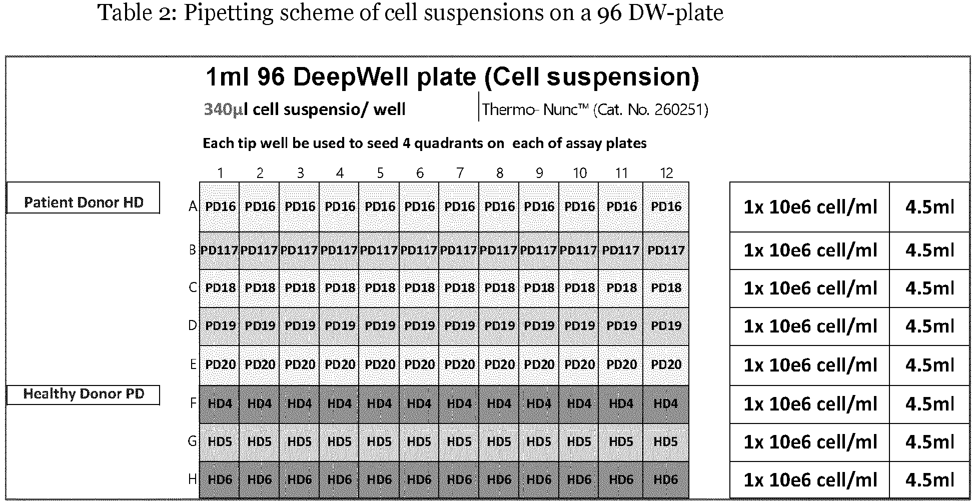

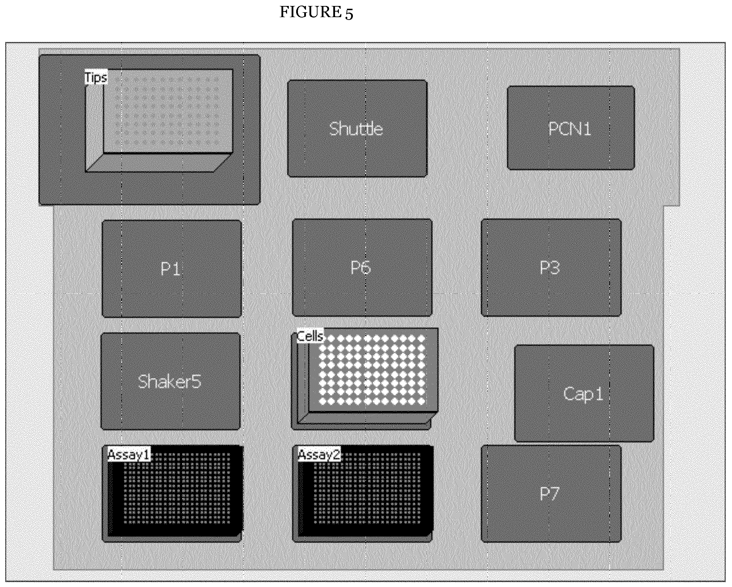

384-DW plate (assay plate 1 and 2) and 96-DW plate were placed on the worktable of a pipetting robot as shown in Figure 5. Each tip (of 96 heads) of a pipetting robot was loaded with 170 µl of cell suspension from the 96 DW-plate and 40µl were pipetted into each of the quadrants on assay plate 1 and 2. Then the remaining 10 µl were pipetted into the source plate (96-DW plate). 20 µl of the LPS stock solution 1 (with a concentration of 60 ng/ml) were transferred to the 40 µl of seeded PBMC cells in the 384-DW plate to obtain a final concentration of 20 ng/ml. This was followed by a 2-hour incubation of the 384-DW plate at 37°C, whereby the inflammasomes of the cells in the 2-hour incubated plate were subsequently activated with Nigericin, and a 2.5-hour incubation of the 384-DW plate, whereby the inflammasomes of the cells in the 2.5-hour incubated plate were subsequently activated with Leu Leu.

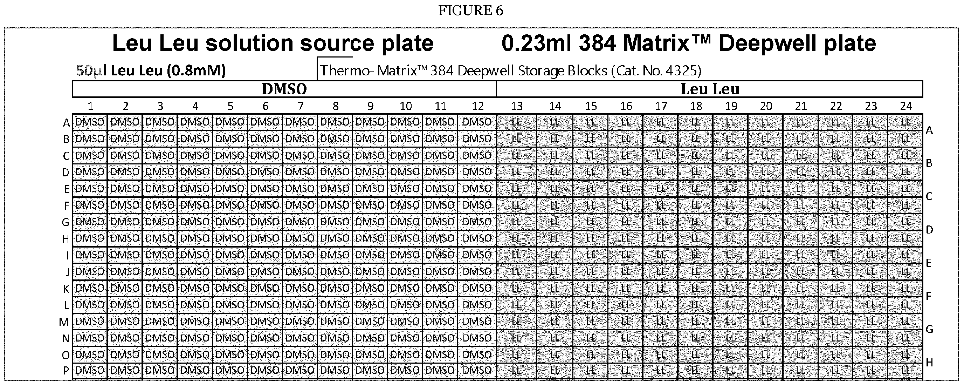

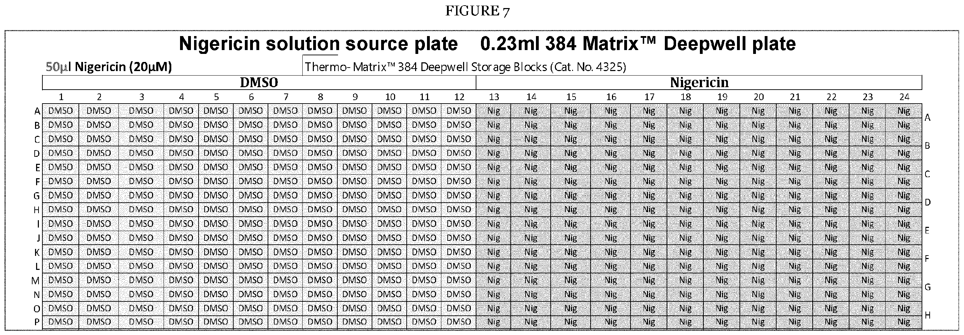

Inflammasome activation: Leu Leu and Nigericin source plates were provided for inflammasome activation of the PBMCs. Therefore, a 1 M Leu Leu stock solution (Leu-Leu-OMe·HCl ChemImpex; Cat.No. 04578, Mw 294.82; LOT number: 000424-130420-11834) in DMSO was adjusted to a Leu Leu source solution with a final concentration of 0.8 mM (working dilutions in DPBS w/o Ca2+/Mg2+, containing 25mM HEPES (Gibco, Cat. No. 15630056, Lot. No. 1742638)) and a DMSO source solution (DMSO, AppliChem Cat. No. 3672,0100; 1 M HEPES, PBS) was provided as a control, wherein both source solutions were pipetted according to the pipetting scheme shown in Figure 6. A Nigericin stock solution with a concentration of 10 mM (Life Technologies, Cat.No. N-1495; 10 mg; Mw 724.97; LOT number: 1800830) was adjusted to a final concentration of 20 µM in complete medium and a DMSO solution was also provided as a control, wherein both source solutions were pipetted according to the pipetting scheme shown in Figure 7.

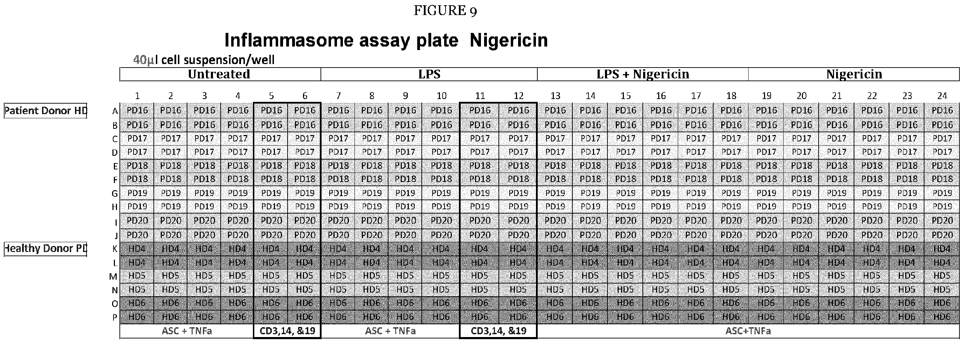

20 µl of the provided 20µM Nigericin solution were pipetted per well onto the Nigericin plate incubated with LPS for 2 hours (5 µM final on cells) or DMSO control from the Nigericin source DW plate, followed by incubation at 37°C for one hour as shown in Figure 9.

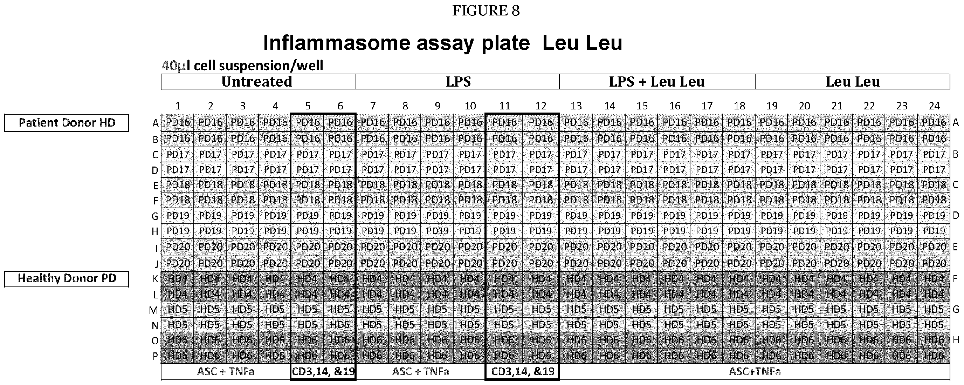

20µl of the provided 0.8 mM Leu Leu solution were pipetted per well onto the Leu Leu plate incubated with LPS for 2.5 hours (0.2 mM final on cells) or DMSO control from the Leu Leu source DW plate, followed by incubation at 37°C for 0.5 hour as shown in Figure 8.

Supernatant collection and cell fixation: 30µl of the supernatant from each well of each assay plate were collected twice on collection plates for further investigation. 20 µl per well of an 8% PFA solution (16% Formaldehyde (w/v), meth-free, Thermo, Cat. No. 28908; 10ml of 16% Formaldehyde (w/v, meth-free); 10ml DPBS, no Ca2+, no Mg2+ (Life Technologies, Cat. No. 14190-169) were added to the assay plates and incubated for 15 minutes fixation time at RT. In the meantime, the collection plates were centrifuged at 350 x g for 2 min. Then 8 µl were transferred from the collection plate to each of the 4 HTRF plates and 16µl from collection plate 1 to storage plate 1. HTRF assay plates and storage plates were sealed and frozen for later investigations. The cell fixation was finished by removing the fixation solution (2 x 20µl) and adding 30 µl of FBS/PBS solution (5 ml of Hi-FBS (Sigma, Cat. No. F9665, Charge 065M3367) to 500ml DPBS, no Ca2+, no Mg2+ (Life Technologies, Cat. No. 14190-169)).

Immunocytochemistry assay: The following solutions were prepared.

**For Cell Surface markers (CDs) staining:**
- CDs Blocking Buffer (1% BSA/PBS: 5 g of Bovine Serum Albumin (BSA, low endotoxin, lyophilized powder, Sigma Cat. No. A1470, Lot# SLBNi886v) were dissolved in 500ml DPBS, no Ca2+, no Mg2+ (Life Technologies, Cat. No. 14190-169))
- commercially available Hoechst33342 stock solution (10mg/ml): (BisBenzimide H33342 trihydrochloride, H33342, ≥98% (HPLC and TLC), Sigma-Aldrich, Cat. No. B2261, 100mg; 10mg of H33341 in were dissolved in 1000 µl DMSO (Dimethyl Sulfoxide, for cell culture, AppliChem, Cat.No. A3672,0100) to reach a stock concentration of 10mg/ml) to Hoechst dilution 1 : 2500 with a final concentration of 4 µg/ml; Hoechst33342 is a nucleic acid dye with blue fluorescence when bound to dsDNA.
- CDs staining solution (10 µl CD3 (Alexa Fluor® 488 anti-human CD3 Antibody; 1:500 (0.4 µg/ml)) + 100µl CD14 (PE Mouse Anti-Human CD14; 1:50 (0.24 µg/ml)) + 50 µl CD19 (Alexa Fluor® 647 anti-human CD19 Antibody; 1:100 (0.5 µg/ml)) + 5 ml Hoechst/CDs Blocking Buffer).

**For ASC- TNFα staining:**
- ASC Blocking-Permeabilization Buffer (0.5% Triton Tx-100; 1%BSA/PBS: 2.5ml Triton™ X-100 (molecular biology, Sigma, Cat. No.T8787, Lot#036k00851) were dissolved in 500ml 1% BSA/FBS solution)
- Hoechst dilution 1:10000 (final 1 µg/ml) for ASC staining
- ASC-TNFα staining solution (ASC 1:500: 40 µl ASC (rabbit anti-PYCARD-DY488; 1:500 (1.1 µg/ml)) + 400µl TNFa (TNF-α (D1G2) Rabbit mAb; 1:50 (0.12 µg/ml)) + 20ml Hoechst/ASC Blocking Buffer)

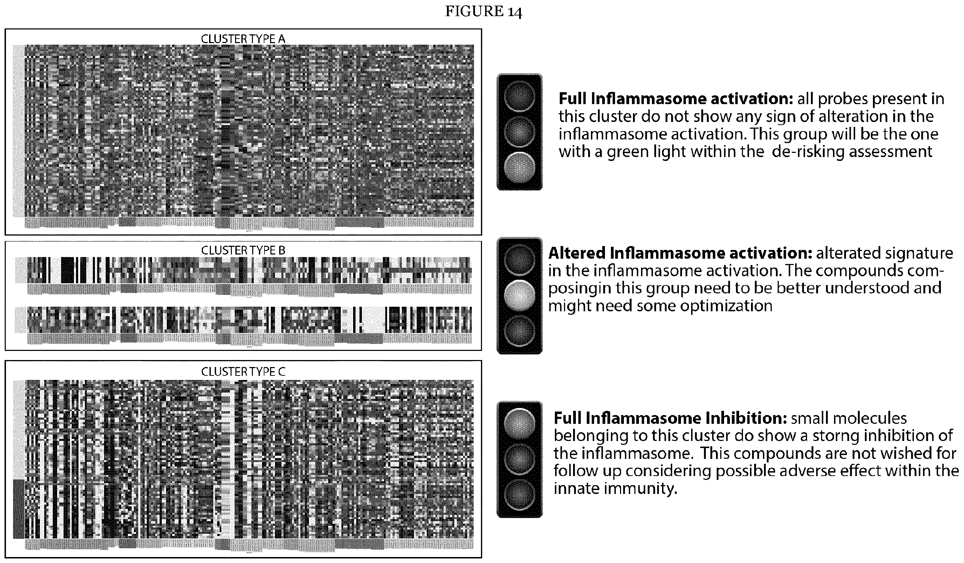

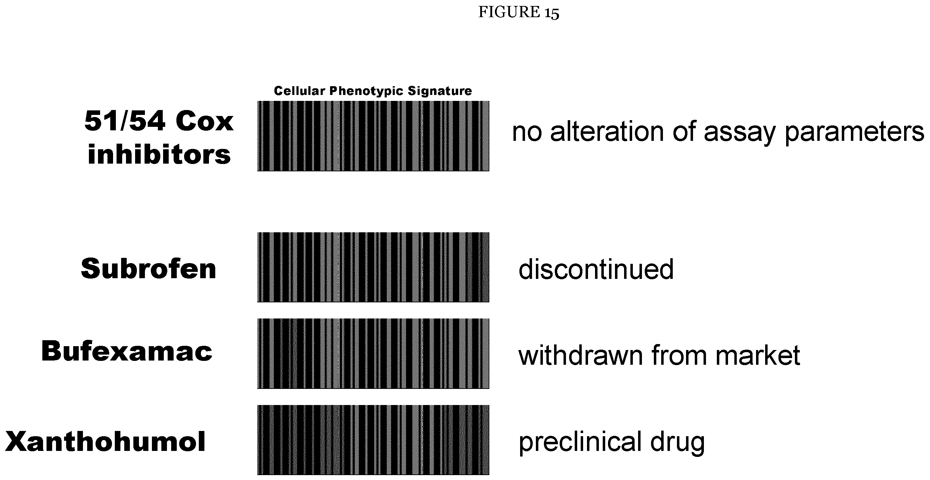

**Loading of 384 DW plates:** A 384 DW plate was loaded with either 50 µl per well of ASC blocking pre-stabilization buffer or 50µl per well CD blocking buffer according to the pipetting scheme shown in Figure 14. In addition, another 384 DW plate was loaded with either 50µl per well of ASC-TNFa or CD staining solution according to the pipetting scheme shown in Figure 15.

**Staining:** FBS/PBS solution was removed from the assay plates and 20 µl Blocking Buffer per well was added. 2-minute incubation at RT followed. Blocking Buffer was removed from the assay plates and 20µl staining solution was added per well. A further 1 hour incubation at RT in the dark followed. Washing: After incubation the staining solution was removed and washed by adding 20µl FBS/PBS solution per well.

**Image acquisition setting:** The plate was analyzed in two runs.
Acquisition E01: Analysis of ASC-TNFa stained wells
Acquisition E02: Analysis of CD markers stained wells

Hoechst33342; blue fluorescence dye bound to dsDNA (Ch.1): 405nm ET:100ms EG:100; ASC (Ch.2): 488nm ET:100ms EG:100; CD14 (Ch.3): 568nm ET:300ms EG:100; TNFα-CD19(Ch.4): 647nm, ET:250ms EG:120; CD3 (Ch.5): 488nm ET:200ms EG:100. 32 fields were imaged for each well as shown in Figure 16.

Further Inflammasome activators may include the following:
**Adenosine 5-triphosphate disodium salt hydrate** (ATP, A6419-5G, Sigma, 0.5M NaOH (Roth Cat.No. 9356.1, MW 40.01g/mol) in sterile milliQ H2O; 500 mM ATP (A6419-5G, MW 551.14, anhydrous basis): 275.57 mg/ml in 0.5M NaOH; Working dilutions in DPBS w/o Ca2+/Mg2+, containing 25mM HEPES)
**Monosodium Urate Crystals (MSU crystals)** (InvivoGen, Cat. No. tlrl-msu, 5 mg; Preparation of MSU stock solution (5mg/ml): Dissolve 5 mg MSU in 1 ml sterile DPBS w/o Ca2+/Mg2+; Working dilutions in sterile DPBS w/o Ca2+/Mg2+; MSU crystals final concentration range: 250 µg/ml - 500 µg/ml.
**Alum Crystals** (InvivoGen, Cat. No. tlrl-alk, 1g; Alum crystal stock solution (20mg/ml = 40mM): 100mg Alum crystals in 5ml sterile endotoxin-free H2O (WFI, LifeTechnologies, Cat. No. A12873-01); Working dilutions in DPBS (w/o Ca2+/Mg2+), Alum crystals final concentration range: 100 µg/ml - 200 µg/ml final.
**Silica Crystals** - **Nano-SiO2** (InvivoGen, Cat. No. tlrl-si, 10mg; Preparation of Silica crystal stock solution (5mg/ml, i.e. 83 mM): Dissolve 10mg Nano-SiO2 crystals in 2 ml sterile endotoxin-free H2O (WFI, LifeTechnologies, Cat. No. A12873-01); Working dilutions in complete media; Nano-SiO2 final concentration range: 10 µg/ml - 200 µg/ml)
**CPPD crystals** - **Calcium pyrophosphate dehydrate crystals** (InvivoGen, Cat. No. tlrl-cppd, 5mg; Preparation of Silica crystal stock solution (5mg/ml): Dissolve 5mg Nano-SiO2 crystals in 1 ml sterile DPBS w/o Ca2+/Mg2+; Mix by vortexing and sonicate suspension for 5 minutes; Store at 4°C (short-term or -2°C (long-term); Working dilutions in sterile DPBS w/o Ca2+/Mg2+; CPPD crystals final concentration range: 1µg/ml - 50 µg/ml.
**Cholesterol crystals** (Cholesterol, Sigma, Cat. No. C8667; Dissolve 12.5mg/ml cholesterol in 95% ethanol)
**Poly(dA:dT)** (Poly(dA:dT), InvivoGen, Cat. No. tlrl-patn-1, 1mg; Dissolve 1mg poly(dA:dT) in 1ml endotoxin-free H2O; Stimulate cells with 10ng/ml - 10µg/ml poly(dA:dT) complex for 6-24h)

### Nanoparticles/Nanoplastic

### Example 2:

### De-risking approach for Cox-Inhibitors (painkiller)

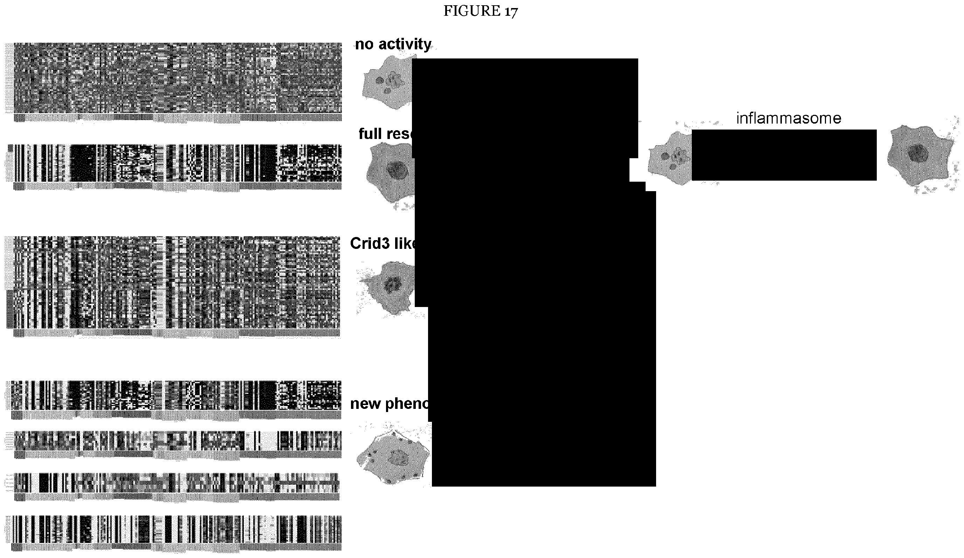

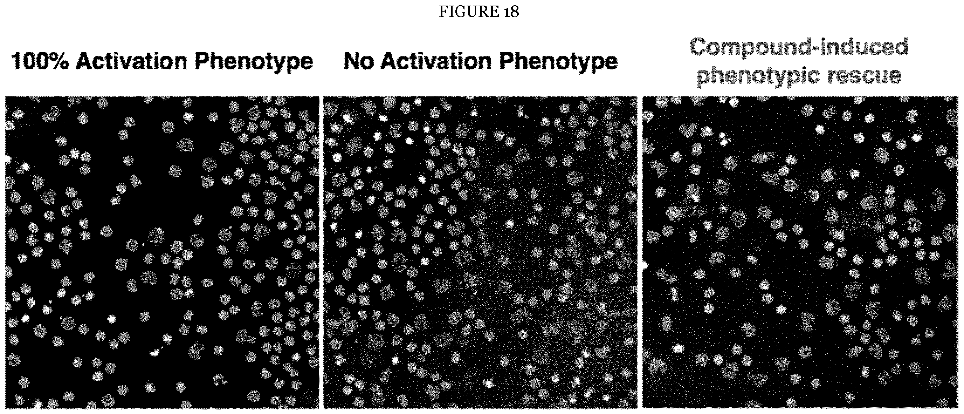

54 compounds were tested, which are known as cyclooxygenase (COX)-Modulators. Most of the compounds were already on market or in drug development process. A unique collective phenotypic signature of this drug class was identified. There was no alteration of assay parameters, meaning that common Cox-modulators should have no effect on the innate immune response in the test system provided by the present invention. Surprisingly, three compounds (Subrofen, Bufexamac and Xanthohumol) were identified to show alteration of the collective phenotypic signature. Subrofen, an early nonsteroidal anti-inflammatory drug (NSAID), was discontinued due to adverse side effects and showed a strong decrease of cytokine release (ILib) in the test system of the present invention. Bufexamac was withdrawn from market 2010 due to allergic reaction and was further proposed in 2017 as co-treatment agent (TNFa binder) for modulators of TNFa synthesis. This effect was proofed by using the de-risking approach of the present invention. Xanthohumol, a chalcone isolated from Humulus lupus, is currently in clinical trial phase 1. Currently, there is no information about induced adverse effects. The results are shown in Figures 17 and 18.

The adverse effects of all three compounds were not identified during the safety/toxicity assessment in preclinical lead optimization or in clinical trials. Current safety testing during preclinical drug development was not able to detect the adverse modulation of innate immune response. These results show the potential of the present invention and de-risk drugs, drug candidates, cosmetics or materials (e.g. nanomaterial) humans are unwillingly exposed to.

### Example 3:

### Stratifying of patients suffering from an inflammation-related disease and healthy volunteers

The inventive assay was performed using hPBMC samples from 3 healthy volunteers and hPBMC samples from 20 patients suffering from an inflammation-related disease, in particular patients suffering from Alzheimer's disease, depression, and other dementia diseases. The assay was performed automatically as described above. It was shown that the inflammasome responses observed in hPBMCs obtained from patients suffering from an inflammation-related disease, in particular suffering from a neurodegenerative disease, were significantly altered compared to the responses of hPBMCs obtained from healthy humans, which allowed the classification into disease groups. The results are represented in Figure 19.

## Claims

1. An *in vitro* method for assigning a phenotypic signature of cells in a liquid biological sample obtained from a mammal, to an innate immune response group, said method comprising:
i) Stimulating said cells in said biological sample comprising cells with an inducing or repressing agent of the innate immune response;
ii) Separating said cells as stimulated from at least part of the supernatant of said biological sample;
iii) Collecting at least part of said supernatant, and analysing said supernatant for factors that induce or repress the innate immunity as released from said cells;
iv) Suitably staining of microscopically evaluable intracellular and/or extracellular structures of said stimulated cells from step ii), and subsequent microscopic evaluation of said stained intracellular and/or extracellular structures;
v) Analysing both of said factors as released into the supernatant and said microscopic evaluation in order to obtain a combined phenotypic signature indicating the innate immune response group of said cells; and
vi) Assigning said phenotypic signature to an innate immune response group comprising the use of multivariate classification algorithms.

2. An *in vitro* method for identifying whether a mammal suffers from an inflammation-related disease or is at risk of suffering from an inflammation-related disease, said method comprising:
i) Providing a liquid biological sample obtained from said mammal comprising cells,
ii) Stimulating said cells in said biological sample with an inducing or repressing agent of the innate immune response;
iii) Separating said cells as stimulated from at least part of the supernatant of said biological sample;
iv) Collecting at least part of said supernatant, and analysing said supernatant for factors that induce or repress the innate immunity as released from said cells;
v) Suitably staining of microscopically evaluable intracellular and/or extracellular structures of said stimulated cells from step ii), and subsequent microscopic evaluation of said stained intracellular and/or extracellular structures;
vi) Analysing both of said factors as released into the supernatant and said microscopic evaluation in order to obtain a combined phenotypic signature indicating the innate immune response group of said cells; and
vii) Assigning said phenotypic signature to an innate immune response group comprising the use of multivariate classification algorithms;
wherein a change of said phenotypic signature of said mammal when compared to said phenotypic signature in a healthy mammal identifies a mammal suffering from an inflammation-related disease or being at risk of suffering from an inflammation-related disease.

3. An *in vitro* method for stratifying a mammal suffering from an inflammation-related disease, said method comprising:
i) Providing a liquid biological sample obtained from said mammal comprising cells;
ii) Stimulating said cells in said biological sample with an inducing or repressing agent of the innate immune response;
iii) Separating said cells as stimulated from at least part of the supernatant of said biological sample;
iv) Collecting at least part of said supernatant, and analysing said supernatant for factors that induce or repress the innate immunity as released from said cells;
v) Suitably staining microscopically evaluable intracellular and/or extracellular structures of said stimulated cells from step ii), and subsequent microscopic evaluation of said stained intracellular and/or extracellular structures;
vi) Analysing of both said factors as released into the supernatant and said microscopic evaluation in order to obtain a combined phenotypic signature indicating the innate immune response group of said cells;
vii) Assigning said phenotypic signature to an innate immune response group comprising the use of multivariate classification algorithms;
wherein said mammal is stratified for a treatment against said inflammation-related disease, if the phenotypic signature of said mammal deviates from the same phenotypic signature in a healthy mammal.

4. An *in vitro* method for monitoring the progression of an inflammation-related disease in a mammal being treated against an inflammation-related disease or being at risk of suffering from an inflammation-related disease, comprising:
i) Providing a liquid biological sample obtained from said mammal comprising cells;
ii) Stimulating said cells in said biological sample with an inducing or repressing agent of the innate immune response;
iii) Separating said cells as stimulated from at least part of the supernatant of said biological sample;
iv) Collecting at least part of said supernatant, and analyzing said supernatant for factors that induce or repress the innate immunity as released from said cells;
v) Suitably staining of microscopically evaluable intracellular and/or extracellular structures of said stimulated cells from step ii), and subsequent microscopic evaluation of said stained intracellular and/or extracellular structures;
vi) Analysing of both said factors as released into the supernatant and said microscopic evaluation in order to obtain a combined phenotypic signature indicating the innate immune response group of said cells;
vii) Assigning said phenotypic signature to an innate immune response group comprising the use of multivariate classification algorithms;
wherein, during said treatment of said mammal, the influence of said treatment on the progression of the inflammation-related disease is monitored by a change in said phenotypic signature of said mammal.

5. The method according to any one of claims 1 to 4, wherein said inducing or repressing agent of the innate immune response induces or represses the activity of a factor selected from the group consisting of Toll-like receptors (TLRs) and/or Nod-like receptors (NLRs), such as, for example TLR4/TLR2, NLPR3, AIM2 and/or NLRC4, and is preferably selected from lipopolysaccharide (LPS), LeuLeuOMe, Nigericin, ATP, MSU crystals, aluminium crystals, nano-SiO2, CPPD crystals, cholesterol crystals, Poly(dA:dT), nanoparticles, flagellin, therapeutic antibodies, CAR-T cells, amyloid beta, Tau, alpha-synuclein, TDP43, exosomes, and nanoplastic.

6. An *in vitro* method for identifying a compound and/or environmental condition that induces or represses the innate immune response of cells in a liquid biological sample obtained from a mammal, said method comprises:
i) Contacting said cells in said liquid biological sample with a compound and/or exposing said cells in said liquid biological sample to environmental conditions that potentially induce or repress the innate immune response of said cells;
ii) Separating said cells from at least part of the supernatant of said liquid biological sample;
iii) Collecting at least part of said supernatant, and analysing said supernatant for factors released from said cells that induce or repress the innate immunity;
iv) Suitably staining of microscopically evaluable intracellular and/or extracellular structures of said contacted cells, and subsequent microscopic evaluation of said stained intracellular and/or extracellular structures;
v) Analysing of both said factors as released into the supernatant and said microscopic evaluation in order to obtain a combined phenotypic signature indicating the innate immune response group of said cells, and
vi) Identifying said compound as inducing or repressing said innate immune response based on said phenotypic signature as obtained in step v), using multivariate classification algorithms.

7. The method according to claim 6, wherein said compound is selected from a drug, a small molecule, a protein, a peptide, an antibody or fragment thereof, a polynucleotide, an oligonucleotide, nanoparticles, a carbohydrate, and lipids.

8. The method according to any one of claims 1 to 7, wherein said inflammation-related disease is an autoimmune disease, or preferably a neurodegenerative disease.

9. The method according to any one of claims 1 to 8, wherein said separating comprises immobilizing said cells on a solid carrier material that is transparent, preferably selected from glass or a plastic material, and preferably chemically inert, and wherein preferably in step of analyzing of both said factors as released into the supernatant and said microscopic evaluation in order to obtain a combined phenotypic signature a proteome, transcriptome, lipidome and/or genome of said cells is evaluated, and clinical data are included to supplement said combined phenotypic signature.

10. The method according to any one of claims 1 to 9, wherein said cells are selected from immune cells, peripheral blood mononuclear cells (PBMCs), PBMCs co-cultured with other cells in 2D and 3D culture, lymphocytes and monocytes.

11. The method according to any one of the claims 1 to 10, wherein said microscopically evaluable intracellular and/or extracellular structures of said cells that are stained comprise lipids, sugars, nucleic acids, functional compartments and/or intracellular and/or extracellular proteins, such as for example, PYCARD, TNF-alpha, CD14, CD19, CD3, IL-1-beta, CASP1, NFkappaB, and NLRP3, and wherein preferably said released factors in the supernatant to be analysed comprise cytokines, such as for example, TNF-alpha, and IL-1-beta; and wherein preferably said microscopic evaluation comprises at least one of morphology, subcellular location of said stained intracellular and/or extracellular structures, and marker intensity of said stained intracellular and/or extracellular structures of said cells.

12. The method according to any one of the claims 1 to 11, wherein said evaluation of said phenotypic signature is carried out automatically, and preferably comprises a comparison with a respective control from a database, and wherein preferably said innate immune response group is determined based on changes in the phenotypic signature when compared to a respective control from a database.

13. The method according to any one of claims 1 to 12, wherein said analysis of said innate immune response group comprises the analysis of the inflammasome activation status of said cells in said liquid biological sample.

14. A phenotypic signature as obtained from the method according to any one of claims 1 to 13.

15. Use of a phenotypic signature according to claim 19 for determining the innate immune response group of a mammalian cell, or for identifying whether a mammal suffers from an inflammation-related disease or is at risk of suffering from an inflammation-related disease, or for stratifying a mammal suffering from an inflammation-related disease, or for monitoring the progression of an inflammation-related disease in a mammal, or for identifying a compound and/or environmental condition that induces or represses the innate immune response of mammalian cells.
